# EUROPÄISCHE PATENTSCHRIFT

(11) **EP 0 778 347 B1**
(45) Veröffentlichungstag und Bekanntmachung des Hinweises auf die Patenterteilung: **24.05.2006**
(21) Anmeldenummer: 96118816.6
(22) Anmeldetag: 25.11.1996
(51) Int. Cl.: C12N 15/55, C12N 15/61, C12N 9/14, C12N 9/90, C12Q 1/42, C12Q 1/533, G01N 33/50, C12P 21/02, C12N 15/11

(54) **ATP- und Nukleinsäure-bindendes Protein mit Helikase- und ATPase Eigenschaften**
ATP and nucleic acid-binding protein with helicase and ATPase characteristics
Protéine liant l'ATP et les acides nucléiques et ayant des caractéristiques d'hélicase et d'ATPase

(30) Priorität: 04.12.1995 DE 19545126
(43) Veröffentlichungstag der Anmeldung: 11.06.1997
(73) Patentinhaber: Aventis Pharma Deutschland GmbH, 65929 Frankfurt am Main (DE)
(72) Erfinder: Kirschbaum, Bernd, Dr., 55122 Mainz (DE); Müllner, Stefan, Dr., 65239 Hochheim (DE); Bartlett, Robert, Dr., 64291 Darmstadt (DE)
(74) Vertreter: Ackermann, Joachim

(56) Entgegenhaltungen:
- WO-A-94/23059
- WO-A-94/28157
- US-A- 5 466 576
- DATABASE EMBL - EMEST6 Entry HS24364, Acc.No. T95243, 14.April 1995 HILLIER, L. ET AL.: "ye39a10.r1 Homo sapiens cDNA clone 120090 5' similar to SP:Ko3H1.2 CE01027 RNA helicase." XP002061645
- ONO, Y. ET AL.: "Identification of a putative RNA helicase (HRH1), a human homolog of yeast prp22." MOLECULAR AND CELLULAR BIOLOGY, Bd. 14, Nr. 11, November 1994, Seiten 7611-7620, XP002061642
- ZIELINSKI, T.: "Leflunomide, a reversible inhibitor of pyrimidine biosynthesis?" INFLAMMATION RESEARCH, Bd. 44, Nr. Suppl.2, August 1995, Seiten S207-S208, XP002061643
- DATABASE EMBL - EMHUM1 Entry Hsd977, Acc. No. D86977, 25.August 1996 NOMURA, N.: "Human mRNA for KIAA0224 gene, complete cds." XP002061646 -& NAGASE, T. ET AL.: "Prediction of the coding sequences of unidentified human genes. VI. the coding sequences of 80 new genes (KIAA0201-KIAA0280) deduced by analysis of cDNA clones from cell line KG-1 and brain." DNA RESEARCH, Bd. 3, 1996, Seiten 321321-329, XP002061644

## Beschreibung

Die vorliegende Erfindung hat die Identifizierung und molekularbiologische und biochemische Charakterisierung eines ATP- und Nukleinsäure-bindenden Proteins mit Helikase- und ATPase-Eigenschaften, sowie Verfahren zu dessen Herstellung und Anwendung in pharmakologisch relevanten Testsystemen zum Gegenstand.

Die Modulation der RNA-Struktur ist ein wesentlicher regulatorischer Prozess bei vielen zellulären Vorgängen, wie z.B. prä-mRNA-Splicing, Zusammenbau von Spliceosomen, Zusammenbau von Ribosomen, Proteintranslation, die unter dem Oberbegriff "Regulation der Genexpression auf RNA-Ebene" zusammengefaßt werden können. Die sogenannte "DEAD-Box"-Proteinfamilie von putativen RNA-Helikasen, benannt nach dem charakteristischen Aminosäure-Motiv Asp-Glu-Ala-Asp (im Ein-Buchstabencode DEAD), spielt hierbei (insbesondere für die Modulation der Sekundär- und Tertiärstruktur von mRNA) eine Schlüsselrolle. Obwohl die Mitglieder dieser Familie und einiger Unterfamilien Unterschiede in ihrer spezifischen Funktion und zellulären Lokalisation besitzen, zeigen sie neben charakteristischen Sequenzhomologien auch ähnliche biochemische Eigenschaften (F.V. Fuller-Pace, Trends in Cell Biology, Vol 4, 1994, 271-274). Die charakteristischen Protein-Sequenzen der DEAD-Proteine sind in der Evolution hoch konserviert (S.R. Schmid and P. Lindner, Molecular and Cellular Biology, Vol 11, 1991, 3463-3471). Mitglieder dieser Protein-Familie finden sich in verschiedenen Viren, Bakterien, Hefen, Insekten, Mollusken, niederen Wirbeltieren bishin zu Säugetieren und zeichnen verantwortlich für eine große Zahl von zellulären Funktionen. Die Tatsache, daß schon relativ einfache Organismen wie z.B. die Hefe Saccharomyces cerevisiae zahlreiche Proteine der DEAD-Box-Proteinfamilie und deren Subfamilien exprimieren, deutet daraufhin, daß möglicherweise jedes dieser Proteine zur spezifischen Wechselwirkung mit bestimmten RNA's oder RNA-Familien beiträgt (I. lost and M. Dreyfus, Nature Vol 372, 1994, 193-196). Translationsfaktoren, wie z.B. eIF-4A sowie die am prä-mRNA-Splicing-Prozess beteiligten Proteine erkennen nachgewiesenermaßen spezifische RNA-Zielsequenzen bzw. -strukturen. Dennoch gibt es bisher nur wenig Informationen über Struktur und Aufbau charakteristischer RNA-Sequenzen, welche die DEAD-Proteine zur Erkennung und zur ATPase/RNA-Helikase-Reaktiön benötigen (A. Pause and N. Sonenberg, Current Opinion in Structural Biology Vol 3, 1993, 953-959).

Die DEAD-Box-Protein-Familie ist eine gegenwärtig ständig wachsende Enzymklasse, die an den vielfältigen Reaktionen zur post-transkriptionalen Regulation der Genexpression beteiligt ist. Wegen der hohen Zahl von unterschiedlichen zellulären DEAD-Box-Proteinen ist zu erwarten, daß bestimmten Klassen von Genprodukten, z.B. viralen Proteinen, Heat-shock Proteinen, Antikörper- und MHC-Proteinen, Rezeptoren, RNA's etc., spezifische RNA-Helikasen zugeordnet sind. Diese Tatsache empfiehlt Mitglieder dieser Proteinfamilie als interessante pharmakologische Targets für die Wirkstoffentwicklung.

Zwei der Subklassen der DEAD-Box-Proteine sind die DEAH- (mit einem spezifischen Aminosäureaustausch) und die DEXH-Proteine (mit zwei Aminosäureaustauschen im Hauptmotiv, wobei X für eine beliebige Aminosäure steht) Familien, die auch bei der Replikation, Rekombination, Reparatur und Expression von DNA und RNA Genomen mitwirken (Gorbalenya, A.E., Koonin, E.V., Dochenko, A.P., Blinov, V.M., 1989: Nucleic Acids Res. 17, 4713-4729). Die DEAD-Box-Proteine und deren Subfamilien werden häufig als Helikase-Superfamilie II zusammengefaßt (Koonin, E.V., Gorbalenya, A.E., 1992: FEBS 298, 6-8). Sie teilen allesamt sieben hochkonservierte Regionen. Insgesamt gehören dieser stetig wachsenden Superfamilie II bislang über 70 Mitglieder an.

Eine schematische Darstellung der DEAD, DEAH und DEXH Familien (Schmid, S.R., Lindner P., 1991: Molecular and Cellular Biology 11, 3463-3471) zeigt die Ähnlichkeit zwischen den Familien. (Die Nummern zwischen diesen Regionen zeigen die Abstände in Aminosäuren (AS). X steht für eine beliebige AS. Wo bekannt, wurden den Bereichen Funktionen zugeordnet.)

Das ATPase-Motiv (AXXXXGKT) ist eine aminoterminale konservierte Region und kommt bei den meisten Nucleotid-bindenden Proteinen vor, so auch bei anderen DNA- und RNA-wechselwirkenden Proteinen wie DNAB (Teil des Primosoms), UvrD (Endonuklease), Elongationsfaktor 1 und Transkriptionsterminationsfaktor Rho (Ford M.J., Anton, I.A., Lane, D.P., 1988: Nature 332, 736-738).

Die zweite konservierte Region ist die sogenannte DEAD-Box, bzw. DEAH, DEXH oder DEXX in anderen Familien der Helikasen und Nucleinsäureabhängige ATPasen. Diese Region stellt das ATPase B-Motiv dar. Bei dem Reaktionsmechanismus bindet die erste Asparaginsäure über ein Wassermolekül Mg²⁺ (Pai, E.F., Krengel, U., Petsko, G.A., Gody, R.S., Katsch, W., Wittinghofer, A., 1990: EMBO J. 9, 2351-2359). Mg²⁺ wiederum bildet einen-Komplex mit dem β- und γ-Phosphat des Nucleotids und ist essentiell für die ATPase-Aktivität. Substitutionen der ersten beiden Reste der DEAD-Region in eIF-4A verhindern die ATP-Hydrolyse und die RNA-Helikase Aktivität, nicht aber die ATP-Bindung (Pause, A., Sonenberg, N., 1992: EMBO J. 11, 2643-2654). Die DEAD-Region koppelt außerdem die RNA-Helikase Aktivität mit der ATPase-Aktivität.

Die dritte untersuchte Region ist die SAT-Region (manchmal auch TAT). Durch Mutation in diesem Bereich wird die RNA-Helikase Aktivität unterbunden, andere biochemische Eigenschaften bleiben jedoch erhalten (Pause A. & Sonenberg N., 1992).

Die am weitesten carboxyterminale Region ist die HRIGRXXR-Region, die für die RNA-Bindung und ATP-Hydrolyse erforderlich ist.

Aus den oben aufgeführten Zusammenhängen ergibt sich, daß spezifische RNA-Helikasen attraktive Targets für pharmazeutische Wirkstoffe darstellen. Da auch von bestimmten pathogenen Viren, welche Krankheiten bei Menschen, Tieren oder Pflanzen hervorrufen können, beispielsweise bekannt ist, daß sie ihre eigene RNA-Helikase im Genom tragen, die für die akkurate Replikation benötigt wird (E.V. Koonin, 1991), liegt eine Anwendung auch im Pflanzenschutz nahe (F.V. Fuller-Pace, Trends in Cell Biology, Vol. 4, 1994, 271-274).

Das Isoxazolderivat Leflunomid zeigt entzündungshemmende und immunsuppressive Eigenschaften, ohne eine Schädigung der bestehenden Funktionen des Immunsystems hervorzurufen (HWA486; R.R. Bartlett, G. Campion, P. Musikic, T. Zielinski, H.U. Schorlemmer in: A.L. Lewis and D.E. Furst (editors), Nonsteroidal Anti-inflammatory Drugs, Mechanisms and Clinical Uses; C.C.A. Küchle, G.H. Thoenes, K.H. Langer, H.U. Schorlemmer, R.R. Bartlett, R. Schleyerbach, Transplant Proc. 1991, 23:1083-6; T. Zielinski, H.J. Müller, R.R. Bartlett, Agents Action 1993, 38:C80-2). Viele Aktivitäten, wie die Änderung der Zellaktivierung, der Proliferation, der Differenzierung und der Zellkooperation, die bei Autoimmunkrankheiten beobachtet werden können, werden durch Leflunomid bzw. dessen aktiven Metaboliten, A77 1726, moduliert.

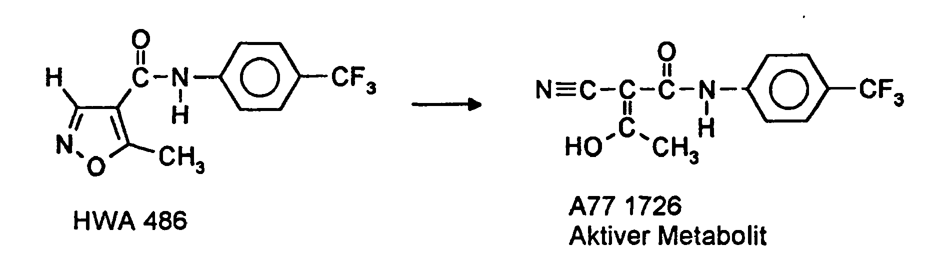

Studien über den molekularen Wirkungmechanismus dieses Wirkstoffs deuten auf einen Einfluß auf den Pyrimidinstoffwechsel hin. Da Leflunomid sehr schnell im Organismus in A77 1726 übergeführt wird, werden beide Bezeichnungen in der vorliegenden Anmeldung praktisch synonym verwendet. Also sind z.B. die Begriffe "Leflunomid-Resistenz" und "A77 1726-Resistenz" sinngemäß identisch.

Pyrimidin- und Purinnukleotide spielen eine Schlüsselrolle in biologischen Prozessen. So sind sie als Bausteine von DNA und RNA Träger der genetischen Information. Die Biosynthese der Pyrimidine beinhaltet die irreversible Oxidation des Dihydroorotats zu Orotat, welche durch das Enzym Dihydroorotat Dehydrogenase (DHODH) katalysiert wird. Insgesamt sechs Enzyme werden für die *de novo* Synthese des Uridin-Mono-Phoshat (UMP) benötigt. UMP spielt eine Schlüsselrolle für die Synthese der anderen Pyrimidine. Cytidin und Thymidin. Die Hemmung der DHODH führt daher zu einer Inhibition der Pyrimidin de novo Synthese. Besonders betroffen sind Immunzellen, die einen sehr hohen Bedarf an Nukleötiden besitzen, aber nur wenig davon durch Seitenwege (salvage pathway) decken können. Bindungsstudien mit radioaktiv markierten Leflunomid-Analoga identifizierten das Enzym DHODH als möglichen Wirkort von A77 1726 und somit stellt die Hemmung der DHODH durch Leflunomid einen wichtigen Ansatz zur Erklärung der beobachteten immunmodulierenden Aktivitäten dar.

Um weitere potentielle intrazelluläre Wirkorte von Leflunomid zu identifizieren, wurde eine Leflunomid-resistente Zellinie entwickelt (siehe auch Beispiel 1).

Diese Resistenz wurde in der hoch proliferativen Zellinie A20.2J (Murine B. Zell Lymphoma) gegenüber A77 1726 induziert. Schrittweise wurde die Konzentration der Substanz in einem serumfreien Kultursystem erhöht, was schließlich zur Etablierung einer stabilen Sublinie namens A20R führte. Die A20R Zellinie toleriert 30-40mal höhere Leflunomidkonzentrationen als die ursprüngliche Zellinie A20.2J (ED₅₀ 130µM gegenüber 4µM).

Überraschenderweise wurde nun gefunden, daß durch eine solche Behandlung einer Leukozytenzellinie mit steigenden, aber nicht-toxischen Dosen des antiproliferativen Wirkstoffes Leflunomid die Expression einer bislang unbekannten putativen RNA-Helikase stimuliert wird, welche der Zelle vermutlich durch effizientere Nutzung vorhandener Transkripte ermöglicht, trotz Substanzeinfluß zu proliferieren. Die vorliegende Erfindung hatte daher zum Gegenstand, die später als DEAH-Box Protein charakterisierte RNA-Helikase durch wirkstoffbedingten Selektionsdruck in einer geeigneten Zellinie zu generieren, das entsprechende Protein zu identifizieren, monoklonale und polyklonale Antikörper gegen das Gesamtprotein, Teile des Proteins und durch proteolytischen Verdau bzw. Peptidsynthese erhaltene Peptidsequenzen auf herkömmliche und literaturbekannte Art herzustellen, einen Reinigungsprozeß für das funktionelle Enzym zu erarbeiten, das Gen oder Genteilsequenzen dieses Proteins nach herkömmlichen und literaturbekannten Methoden zu identifizieren, zu isolieren und zu klonieren, das Gen oder Genteilsequenzen in einem geeigneten Expressionssystem zu exprimieren und sowohl molekularbiologisch, als auch biochemisch strukturell und funktionell zu charakterisieren.

Insbesondere ist es Ziel der vorliegenden Erfindung neue Helikasen und Helikasencodierende Gene bereitzustellen, was mit isolierten DNA-Sequenzen gemäß Anspruch 1 und mit Nukleinsäure-bindenden Proteinen gemäß Anspruch 7 gelungen ist.

Durch Bereitstellung dieses Proteins und verwandter RNA-Helikasen können neue anticancerogene, anti-atherosklerotische, immunsuppressive, antiinflammatorische, antivirale, antifungale und antibakterielle Wirkstoffe aufgefunden werden. Diese werden zur effizienten Therapie von einer Fülle von Krankheiten wie z.B. die Alzheimersche Krankheit, Krebs, Rheuma, Arthrose, Atherosklerose, Osteoporose, akuten und chronischen Infektionskrankheiten, Autoimmunerkrankungen, Diabetes und nach Organtransplantationen dringend benötigt.

Ein Gegenstand der Erfindung ist daher ein Nukleinsäure-bindendes Protein mit Helikase- und ATPase-Eigenschaften gemäß Anspruch 7, dessen mRNA unter Einfluß von Leflunomid oder ähnlich wirkenden Verbindungen verstärkt exprimiert wird, welches vorzugsweise aus einem Abkömmling der murinen Zellinie A20.2J, stammt, und welches besonders bevorzugt die Aminosäuresequenz gemäß Tabelle 2 oder Teile davon enthält.

Ein weiterer Gegenstand der Erfindung ist die DANN gemäß Anspruch 1, die für das Nukleinsäure-bindende Protein mit Helikase- und ATPase-Eigenschaften oder Teile davon kodiert, insbesondere mit der DNA-Sequenz gemäß Tabelle 2

Ferner ist ein Gegenstand der Erfindung eine DNA, die unter stringenten Bedingungen, insbesondere bei 68°C in ExpressHyb-Lösung (Clontech) an die DNA-Sequenz gemäß Tabelle 2 oder Teilen davon, hybridisiert.

Ein weiterer Gegenstand der Erfindung ist eine DNA, die aufgrund der Degeneriertheit des genetischen Codes von den DNA Molekülen, die in dem vorigen drei Absätzen beschrieben worden sind, verschieden ist, jedoch die Expression der entsprechend mit den DNA-Molekülen, die in den vorigen drei Absätzen beschrieben worden sind, exprimierbaren Proteinen ermöglicht.

Darüberhinaus ist Gegenstand der Erfindung ein Vektor, welcher die für das Nukleinsäure-bindende Protein mit Helikase- und ATPase-Eigenschaften kodierende DNA oder Teile davon enthält, und welcher zur Expression besagten Proteins in einer passenden Wirtszelle, die ein weiterer Gegenstand der Erfindung ist, geeignet ist. Ebenfalls ist ein Antisense-Expressionsvektor Gegenstand der Erfindung, der eine Antisense-RNA exprimiert, die mit der RNA, die aus den oben genannten DNA-Molekülen abgeleitet werden kann, unter zellulären Bedingungen hybridisiert.

Ein weiterer Gegenstand der Erfindung sind Verfahren zur Herstellung des Nukleinsäure-bindenden Proteins mit Helikase- und ATPase-Eigenschaften durch Expression des Proteins mittels der erwähnten Vektoren und Wirtszellen und anschließender Isolierung des Proteins mit gängigen Methoden.

Die Nukleinsäure-bindenden Proteine mit Helikase- und ATPase-Eigenschaften, insbesondere der gentechnisch hergestellten, können z.B. in einem Test- bzw. Assay-System zur Auffindung neuer oder Identifizierung bereits bekannter Substanzen in Bezug auf anticancerogene, anti-atherosklerotische, immunsuppresive, antiinflammatorische, antivirale, antifungale oder antibakterielle Wirkung zur Behandlung der Alzheimerschen Krankheit, Krebs, Rheuma, Arthrose, Atherosklerose, Osteoporose, akuter und chronischer Infektionskrankheiten, Autoimmunerkrankungen, Diabetes oder der Folgen einer Organtransplantation. verwendet werden.
Das besagte Assaystem kann so ausgestaltet werden, daß man ausreichende Mengen des Nukleinsäure-bindenden Proteins mit Helikase- und ATPase-Eigenschaften, das möglicherweise auch RNA oder Proteine, welche die RNA Homoeostase beeinflussen können, binden kann, nach gentechnischen Verfahren exprimiert, das erhaltene Protein kristallisiert, mit gängigen Methoden seine dreidimensionale Struktur aufklärt und dann mit gängigen Methoden des "Molecular Modelling" spezifische Hemmstoffe entwickelt, die vorzugsweise mit dem Nukleinsäure-bindenden Protein mit Helikase- und ATPase-Eigenschaften an dessen ATP-Bindungsstelle, der Substrat-Bindungsstelle oder an einer Stelle wechselwirken, welche diese funktionellen Epitope beeinflußt. Der Test auf RNA-Helikase-Aktivität kann nach dem Fachmann bekannten Methoden durchgeführt werden, z.B. können synthetische Oligoribonucleotide an eine Matrix fixiert und mit komplementären, markierten Oligoribonukleotiden hybridisiert werden, woraufhin das Nukleinsäure-bindende Protein mit Helikase- und ATPase-Eigenschaften in oder nicht in Gegenwart möglicher Modulatoren seiner Helikase-Aktivität eine bestimmte, meßbare Menge der markierten, nicht-matrixfixierten Oligoribonucleotide freisetzen kann. Ein solcher Assay kann auch auf Mikrotiterplatten durchgeführt werden wodurch mit hoher Effizienz eine große Anzahl möglicher Modulatoren auf ihre Wirkung getestet werden können.

Ein anderer Assay für Modulatoren des Nukleinsäure-bindenden Proteins mit Helikase- und ATPase-Eigenschaften ist eine Resistenzvermittlung gegenüber Leflunomid von ansonsten nicht-Leflunomid-resistenten Zellen durch Expression des rekombinanten Nukleinsäure-bindenden Proteins mit Helikase- und ATPase-Eigenschaften, mit anschließender Messung des Einflusses von möglichen Modulatoren des Proteins auf die Überlebensfähigkeit solcher resistent gemachter Zellen in Leflunomid-haltigem Kulturmedium.

Ein weiterer Assay für Modulatoren des Nukleinsäure-bindenden Proteins mit Helikase- und ATPase-Eigenschaften sind ATPase- bzw. Splicing-Tests, bei denen die Beeinflussung der ATPase- bzw. Splicing-Eigenschaften des Nukleinsäure-bindenden Proteins mit Helikase- und ATPase-Eigenschaften durch mögliche Modulatoren getestet wird.

Ein weiterer Gegenstand der Erfindung ist die Verwendung des Nukleinsäure-bindenden Proteins zur Isolierung spezifisch an dieses Protein bindender RNA 's oder zur Ermittlung ihrer Oligoribonukleotidsequenz, vorzugsweise indem man besagtes Protein oder Teile davon an eine Matrix koppelt und die so hergestellte Affinitätsmatrix zur Anreicherung von RNA s, die spezifisch an das gekoppelte Protein oder Teile davon binden, aus RNA-Gemischen benutzt und besonders bevorzugt indem man noch zusätzlich die so angereicherten RNA-Moleküle mit PCR-Linkern versieht, eine PCR-Anreicherung durchführt und die so erhaltenen PCR-Fragmente analysiert.

Ein weiterer Gegenstand der Erfindung ist die Verwendung des Gens für das nukleinsäurebindende Protein mit Helikase- und ATPase-Eigenschaften als Selektionsmarker, vorzugsweise als Bestandteil von Vektoren. Bei diesem Gegenstand der Erfindung macht man sich die Beobachtung zunutze, daß bei einer Southern-Blot Analyse genomischer DNA der A20R-Zellen im Vergleich mit genomischer DNA der A20.2J-Zellen das Gen, welches für das Nukleinsäurebindende Protein mit Helikase- und ATPase-Eigenschaften kodiert, eine Amplifikation erfahren hat. Die am Beispiel dieses Gens beobachtete Genamplifizierung durch Leflunomid bzw. Leflunomidanaloga soll u.a. bei der Selektion von Zellen sowie bei der Gentherapie Anwendung finden. In diese Beispiele fließt sowohl der Befund der relativen Resistenzbildung als auch der Befund der Expressionsverstärkung ein.

Unter Hybridisierung unter stringenten Bedingungen wird in der vorliegenden Anmeldung Hybridisierung bei 68°C, ExpressHyb-Lösung (Clontech) verstanden. Das anschließende Waschen erfolgt wie in Beispiel 6 angegeben.

Ein Expressionsvektor zu einer passenden Wirtszelle ist ein Vektor, der in der entsprechenden Wirtszelle zur heterologen Genexpression und zur Replikation (beides mit hoher Effizienz) befähigt ist, und zwar konstitutiv oder nach Induktion durch gängige Methoden.

Ein Antisense-Expressionsvektor ist ein Vektor, der in einer entsprechenden Wirtszelle (s.o.) eine gewünschte Antisense-RNA exprimiert, und zwar entweder konstitutiv oder nach Induktion durch gängige Methoden.

Die Erfindung wird nun anhand der Figuren, Tabellen und Beispiele näher erläutert, ohne darauf beschränkt zu sein.

Die Figuren und Tabellen sind wie folgt beschrieben:

### Figurenlegenden:

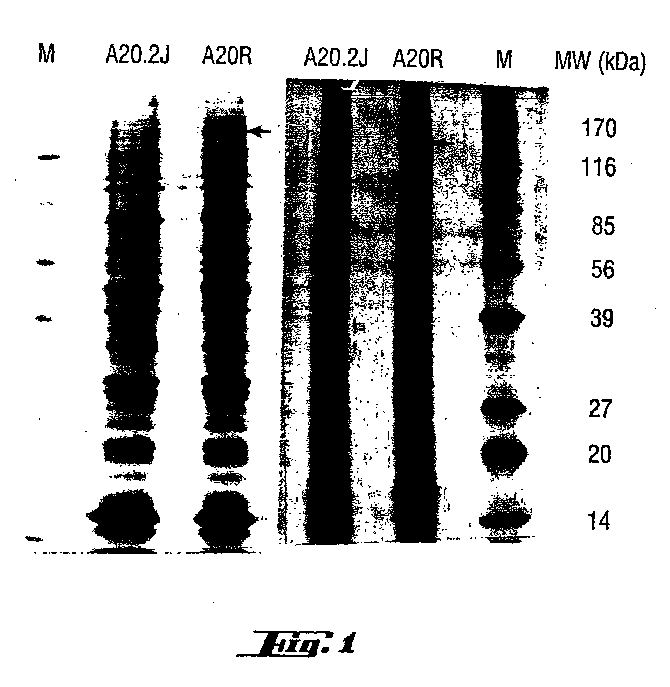

Figur 1: SDS-PAGE (12 % Acrylamid). Die linken drei Gelspuren sind von einem Coomassie-Blau gefärbten Gel, die drei rechten Gelspuren von einem Silber gefärbten Gel. M: Marker (Combithek von Boehringer Mannheim); A20.2J: normale A20-Zellen; A20R: A20-Zellen, die gegen 100 µM Leflunomid resistent sind. Beim Coomassie-Blau gefärbten Gel wurden pro Geltasche jeweils 100 µg Protein, beim Silber gefärbten Gel jeweils 5 µg Protein aufgetragen. Der Pfeil kennzeichnet das Protein, das bei resistenten A20-Zellen verstärkt exprimiert wird.

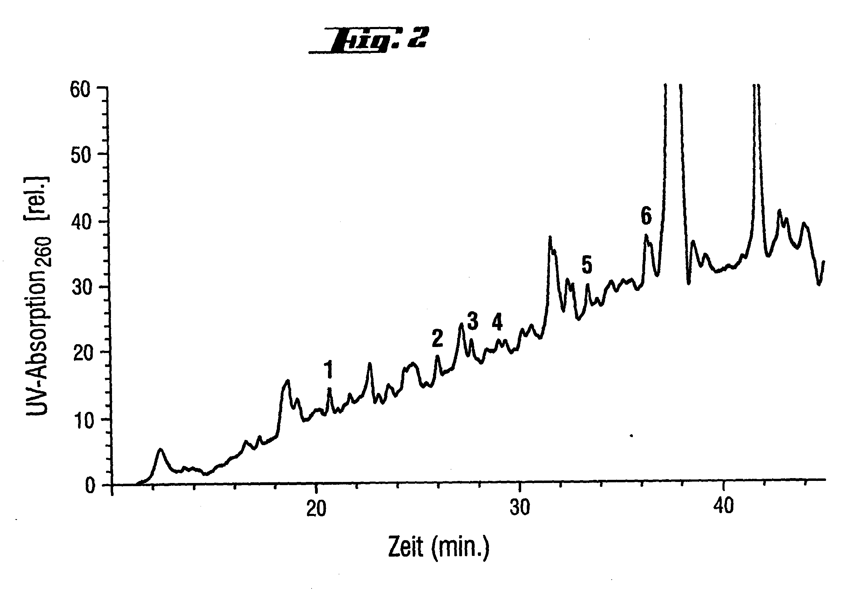

Figur 2: Peptidtrennung durch HPLC. Die HPLC wurde nach den unter Beispiel 1f angegebenen Bedingungen durchgeführt. Im Elutionsprofil sind die 6 Peaks, welche den Peptiden 1-6 des Beispiels 1g entsprechen, fortlaufend durchnumeriert. Auf der Y-Achse sind relative Absorptionseinheiten bei einer Wellenlänge von 206 nm, auf der X-Achse ist die Zeit in Minuten angegeben.

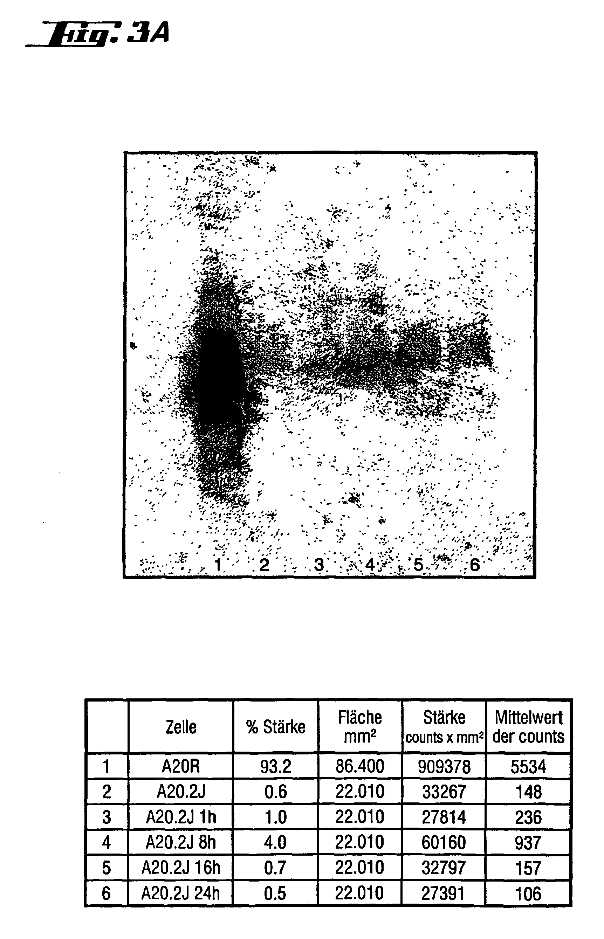

Figur 3: (A) Zeitlicher Verlauf der Expression der putativen RNA-Helikase unter dem Einfluß von Leflunomid bei normalen A20.2J-Zellen im Vergleich zu Leflunomid-resistenten A20R-Zellen. Die Hybridisierung wurde mit der radioaktiv markierten DNA-Sonde A20-5/-6b durchgeführt, deren Sequenz die konservierten Bereiche der DEAD-Box-Protein-DEAH-Subfamilie der putativen RNA-Helikase enthält. Als Molekulargewichtsmarkierung wurde der RNA-Längenstandard I von Boehringer Mannheim verwendet. In der 1. Spur ist die A20R-Gesamt-RNA, in der 2. Spur A20.2J-Gesamt-RNA ohne Behandlung der entsprechenden Zellen mit A77 1726, in der 3.-6. Spur jeweils A20.2J-Gesamt-RNA mit unterschiedlich langer Inkubation der entsprechenden Zellen mit 5 µM A77 1726 (1 Stunde, 8 Stunden, 16 Stunden, 24 Stunden) aufgetragen. Es wurden von jedem Ansatz 20 µg Gesamt-RNA aufgetragen. In Teil (B) ist der gleiche Blot wie unter (A) mit einer β-Actin Probe zur Kontrolle hybridisiert worden.

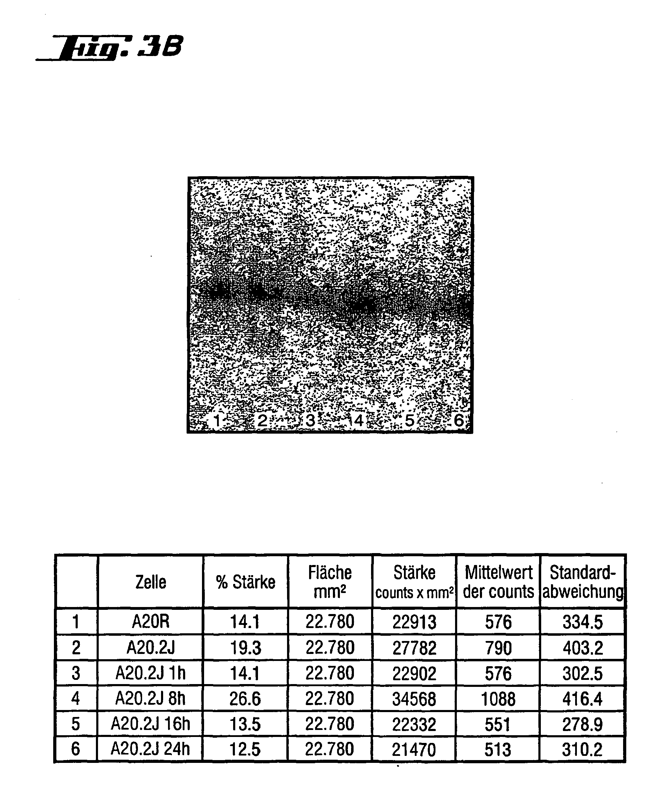

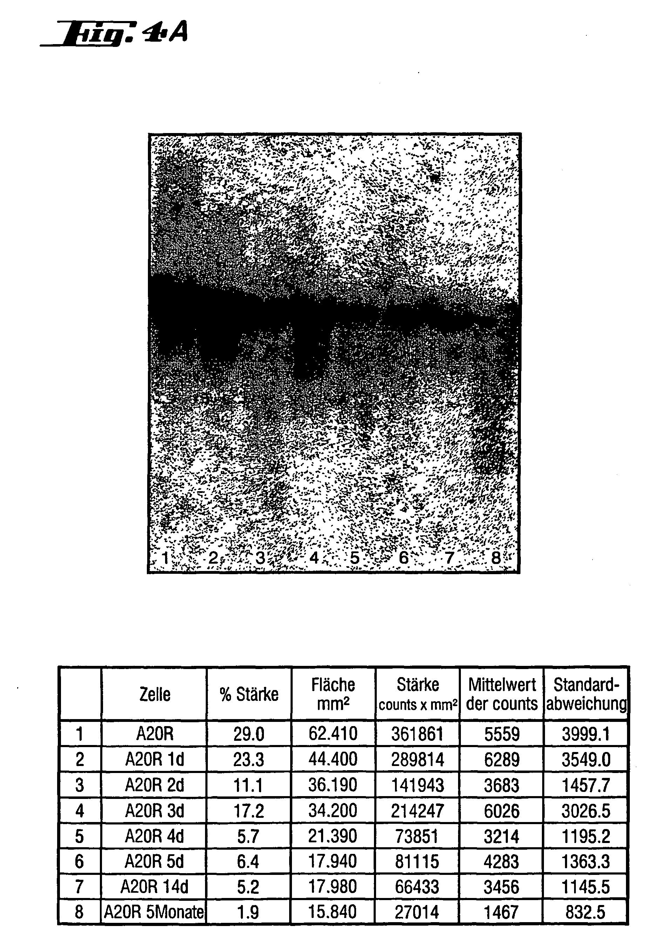

Figur 4: (A) Northern-Experiment zur Expression der putativen RNA-Helikase bei Absetzung von Leflunomid bei Leflunomid-resistenten A20R-Zellen. Die Kontrolle war die RNA von A20R-Zellen, die mit 100 µM Leflunomid inkubiert worden waren (Spur 1). Hybridisiert wurde mit der DNA-Sonde A20-5/6b. Die Spuren 2, 3, 4, 5, 6, 7 und 8 enthielten jeweils 15 µg Gesamt-RNA von A20R-Zellen die ohne Leflunomid über die Zeiträume 1, 2, 3, 4, 5, 14 Tage und 5 Monate inkubiert worden waren. (B) Kontrollhybridisierung des gleichen Blots mit einer β-Actin Probe. Die Blots sind immer mit der entsprechenden quantitativen Auswertung gezeigt.

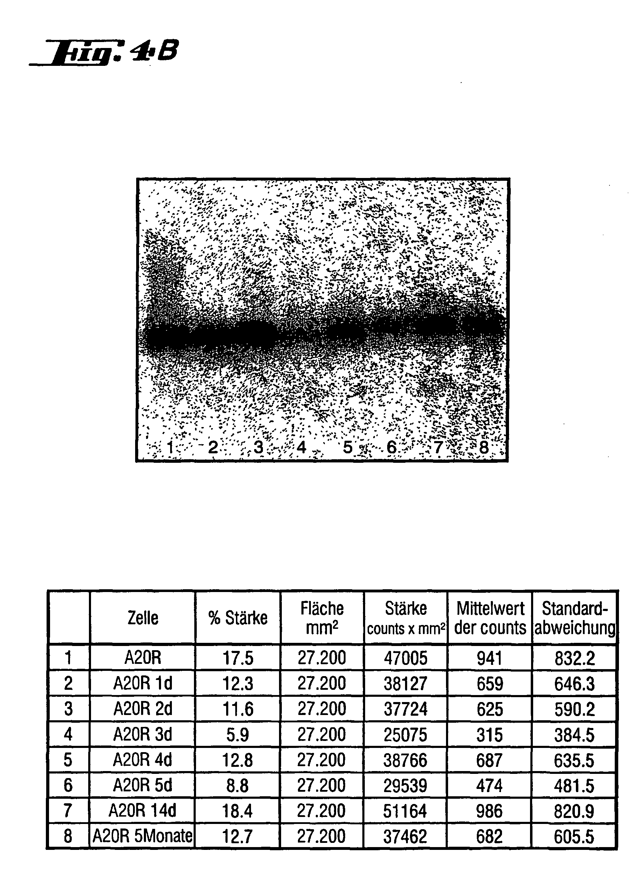

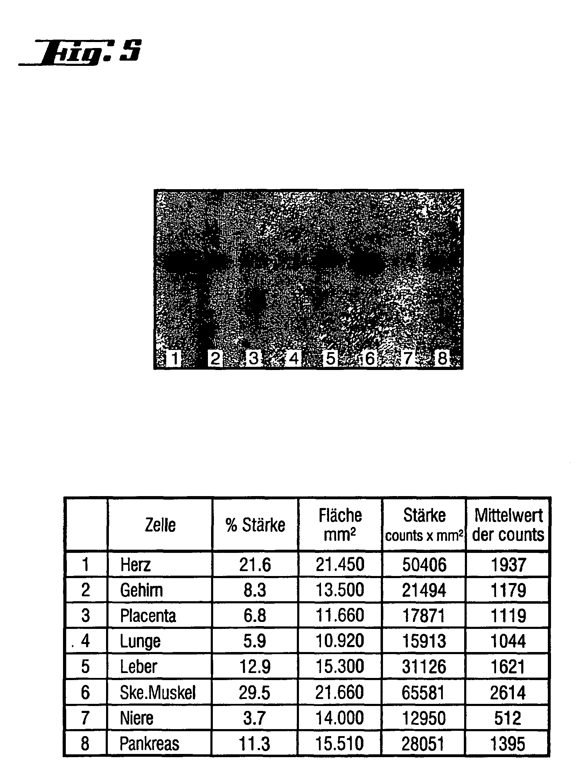

Figur 5: Northern-Blot mit ca. 2 µg Poly (A) RNA pro Spur von acht verschiedenen humanen Geweben. Die Spuren 1-8 enthalten von links nach rechts Gewebe von Herz, Gehirn, Placenta, Lunge, Leber, Skelettmuskel, Niere, und Pankreas. Die RNA wurde auf einem denaturierenden 1,2%igen Agarosegel elektrophoretisch aufgetrennt, und dann auf eine positiv geladene Nylonmembran geblottet, danach durch UV-Quervernetzung fixiert. Hybridisiert wurde mit der A20-5/6b-DNA-Sonde. Die entsprechende quantitative Auswertung ist unter dem Blot gezeigt.

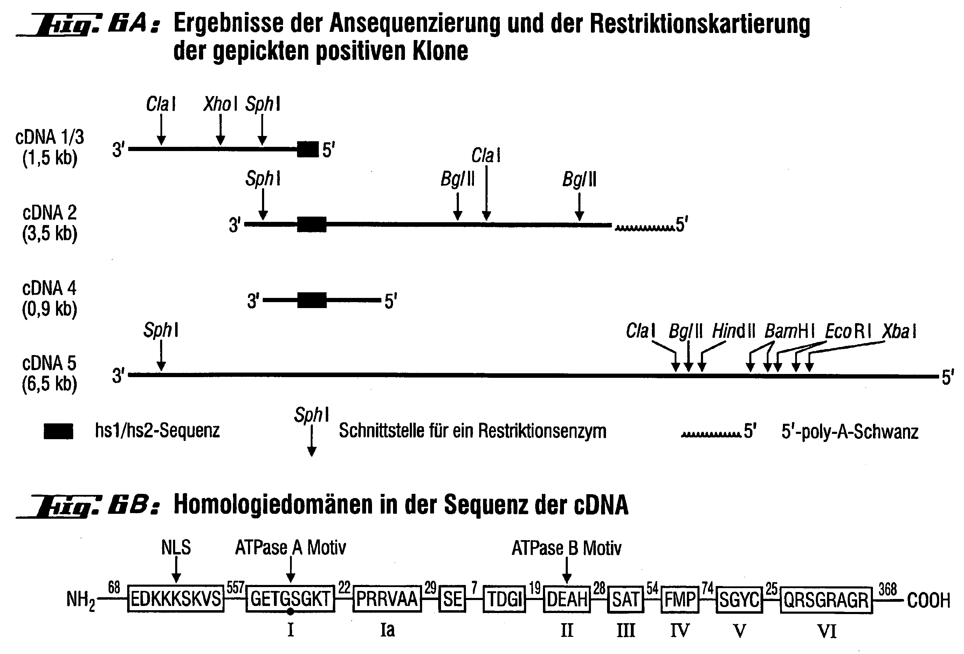

Figur 6: (A) Ergebnisse der Ansequenzierung und der Restriktionskartierung der isolierten positiven Klone. Klone 1 bis 4 überlappen und haben die hs1/hs2-Sequenz im Insert. 1/3 und 2 haben eine Sph I Schnittstelle gemeinsam. cDNA 4 liegt vollständig in cDNA 2. Klon 5 unterscheidet sich durch die Größe (6,5 kb), die Restriktionsschnittstellen und die fehlende hs1/hs2-cDNA von den übrigen Klonen. (B) Homologiedomänen in der Sequenz der cDNA. Die Homologiedomänen sind umrahmt und zwischen den Domänen ist der Abstand in Aminosäuren angegeben. Die Domäne NLS besitzt Homologie zur "nuclear localisation site" aus dem T-Antigen.

Tabelle 1: (A) Primerkonstruktion aus einem Teilbereich des vermehrt exprimierten 135 kDa-Proteins aus A20R. Die jeweils obere Buchstabenreihen kennzeichnen die Aminosäuren im Ein-Buchstaben-Code, darunter ist die Nukleotidsequenz angegeben. Die in Klammern geschriebenen Aminosäuresequenzen sind beginnend mit ihren C-terminalen Ende aufgeführt und leiten sich von DNA-Sequenzen ab, die komplementär zu den hier angegebenen Primersequenzen sind. Es ist jeweils der degenerierte genetische Code angegeben. Da die dritte Base des Codons häufig nicht eindeutig ist, wurde, um in jedem Falle die zugehörige Base für die entsprechende AS zu erhalten, ein Gemisch aller in Frage kommenden Basen synthetisiert. N ist die Abkürzung für alle vier Basen (G, A, T, C). I ist die Abkürzung für Inosin, welches Basenpaarungen sowohl mit Purin- als auch Pyrimidinbasen eingeht. R = A, G; Y = T, C; S = G, C. Bei A20-2, A20-3, A20-4 und A20-5 handelt es sich um stromaufwärts gelegene, degenerierte Primer. A20-6a und A20-6b sind stromabwärts gelegene Primer. Der mittlere Abstand der stromauf- und stromabwärts gelegenen Primer zueinander beträgt etwa 600 Nukleotide. Bei dem unter 6 angegebenen Primer A-20-6b wurde versehentlich das 16. Nukleotid gleich N gesetzt, so daß hier im entsprechenden komplementären Strang sowohl für Isoleucin (ATT, ATC, ATA) als auch für Methionin (ATG) kodiert wird. Diese Tatsache berührte nicht den Erfolg der durchgeführten PCR, jedoch erscheint auf diese Weise fälschlicherweise ein Methionin als sechstletzte Aminosäure in der Sequenz gemäß Tabelle 2 und nicht das korrekte Isoleucin. (B) Primer, abgeleitet aus dem humanen cDNA-Klon B 185; 7 = Stromabwärts-Primer; 8 = Stromaufwärts-Primer.
Tabelle 2: Sequenzierung des Teilbereichs der putativen RNA-Helikase von Leflunomid-resistenten A20R-Zellen. Unterhalb der Basensequenz (1-612) ist die entsprechende Aminosäuresequenz im Einbuchstabencode angegeben. Korrekt ist als sechstletzte Aminosäure Isoleucin und nicht Methionin; ErKlärung siehe Figurenlegende zu Tabelle 1. Das gezeigte DNA-Fragment wurde als A20-5/-6b Sonde für die Hybridisierungsexperimente verwendet.
Tabelle 3: Sequenz des kodierenden Bereiches der Gesamt-cDNA (4272 bp Gesamtlänge). Aus der Lage der Homologien zur Maus-Sequenz ergab sich, daß das erste Leseraster korrekt war. Die kodierenden Sequenzen liegen zwischen Position 148 und 3831 und ergeben eine Sequenz von 1227 Aminosäuren. (* =Stop)
Tabelle 4: Ähnlichkeiten des gefundenen humanen Proteins zu DEAH-Box Proteinen.

### Beispiel 1: Herstellung der Leflunomid-resistenten Zellen

### Medium:

Die Kultivierung der Ausgangslinie, die Züchtung der resistenten Sublinie A20R sowie die Proliferationstests zur Überprüfung der Kreuzresistenz der A20R-Zellen wurden in einem selbsthergestellten, serumfreien Medium vorgenommen. Trockenmedium für 10 Liter Iscove-Medium (Biochrom, Berlin) wurde in 10 Liter bidest. Wasser gelöst. Der Lösung wurden anschließend:
13,95 g NaCl
11,43 g NaHCO₃
700 mg KCl
10 ml 35%ige NaOH-Lösung
0,5 ml 1 molare Mercaptoethanollösung
zugegeben und das Medium sterilfiltriert (alle Substanzen von Riedel de Häen). Vor Gebrauch wurde einem Liter Iscove Medium:
32 mg humanes holo-Transferrin
1 g bovines Albumin
1,5 ml Lipide
(alle Substanzen von Sigma) zugegeben.

### Beschreibung der Ausgangslinie A20.2J:

A20.2J ist eine Sublinie des Maus B - Zellymphomes A20 (ATCC TIB-208) und als Fusionslinie bei der ATCC beschrieben für die Zellinie LS 102.9 (ATCC HB-97). Die Zellen zeichneten sich durch eine hohe Proliferation (Verdopplungszeit ca. 10 Stunden) und einer hohen Sensitivität (50 % Hemmung der Proliferation der Zellen bei 2 µM Substanz) gegenüber A77 1726 (dem Hauptmetaboliten von Leflunomid) aus. Als nichtadhärent wachsende Zellinie waren die Zellen leicht zu kultivieren.

### Beschreibung der Resistenzzüchtung:

A20.2J Zellen wurden anfangs für 5 Tage in Iscove-Medium mit 1 µM A77 1726 kultiviert (Konzentration unterhalb der 50 % Hemmung der Proliferation) und das Zellwachstum sowie die Vitalität der Zellen überprüft. Jeden 2. bzw. 3. Tag wurden die Zellen in frisches Medium passagiert, dem die gleiche Konzentration A77 1726 zugegeben war. War nach 5 Tagen Kultivierung ein Wachstum der Zellen und keine hohe Absterberate (maximal 30 % tote Zellen) erkennbar, so wurde die Konzentration an A77 1726 schrittweise erhöht. Stagnierte die Proliferation der Zellen, so wurde die Konzentration der letzten Passage verwendet. Nach einem Jahr Kultivierung war eine stabile, resistente Sublinie A20R etabliert, die in Gegenwart von 100 µM A77 1726 konstante Proliferation und morphologisch keine Unterschiede zur Ausgangslinie A20.2J zeigte.

### Proliferationsnachweis:

5•10⁵ Zellen wurden in 5 ml lscove-Medium in 6-well-Platten (Greiner) für 48 Stunden bei 37°C und 10 % CO₂ inkubiert.
Ein weil wurde als positiver Bezugswert angesetzt:
- Für die A20.2J : Zellen in Iscove-Medium
- für die A20R : Zellen in Iscove-Medium + 100 µM A77 1726 Zu den Zellen der restlichen wells wurden Testsubstanzen in verschiedenen Konzentrationen pipettiert. Nach der Inkubationszeit wurden die Zellen im weil resuspendiert, 100 µl Zellsuspension entnommen und in 1 %iger Eosinlösung (1 g Eosin yellowish von Riedel de Häen in 100 ml steriler isotonischer Kochsalzlösung gelöst) verdünnt. Die Zellen wurden in einer Neubauer-Zählkammer ausgezählt und der Anteil der toten Zellen (durch Eosin angefärbt) ermittelt. Die substanzinduzierte Veränderung der Proliferation wurde zur jeweiligen Positivkontrolle berechnet.

### Test 2:

4•10³ Zellen wurden in einem Volumen von 100 µl Iscove-Medium in 96-well-Rundbodenmikrotiterplatten (Nunc) pipettiert. Testsubstanzen wurden von der gewünschten Testkonzentration ausgehend doppelt konzentriert angesetzt und 100 µl dieser Lösung zu den Zellen pipettiert. Die Platten wurden für 48 Stunden bei 37°C und 10 % CO₂ inkubiert. Die Proliferation wurde durch radioaktive Markierung der DNA sich teilender Zellen bestimmt. Dazu wurde nach der Inkubationszeit jedem weil 25 µl ³H-Thymidin (10 µCi/ml; spezifische Aktivität 29 Ci/mMol; Fa. Amersham) zugegeben und für weitere 16 Stunden inkubiert. Zur Auswertung des Testes wurden Zellen auf den Platten mittels eines Zellerntegerätes (Skatron) auf Glasfaserfilter (Pharmacia) geerntet, wobei nicht eingebautes ³H-Thymidin in gesonderten Abfallflaschen aufgefangen, und nur zellulär, DNA- gebundene Radioaktivität gemessen wurde. Die Filter wurden in Plastiktaschen verschweißt und nach Zugabe von 10 ml Szintillator (Pharmacia) in Zählkassetten zur Messung verschlossen. Die Messung erfolgte in einem Beta-Counter (Beta-Plate-System 1206 der Firma Wallac). Wie unter Test 1 angegeben wurde die Proliferationsänderung der Testsubstanzen gegen die jeweiligen Positivkontrollen berechnet.

### Beispiel 2: Test der Resistenz der A20R-Zellen

1. Kreuzresistenz gegen literaturbekannte antiproliferative Substanzen: Diverse literaturbekannte antiproliferative Substanzen wurden in verschiedenen Konzentrationen (wie in Proliferationstest 2 beschrieben) auf ihre antiproliferative Eigenschaft auf A20R-Zellen und A20.2J getestet. In der nachfolgenden Tabelle wird die berechnete Hemmung einer Konzentration dieser Substanzen auf beide Zellinien dargestellt. Im Vergleich zur Ausgangslinie A20.2J sollte dargestellt werden, ob bei den A20R-Zellen eine generell höhere Resistenz gegenüber antiproliferativen Substanzen vorlag.

| Testsubstanzen | | % Hemmung der A20.2J | % Hemmung der A20R |
|---|---|---|---|
| Methotrexat | (0,15 µM) | 75,9 | 65,2 |
| Cisplatin | (10 µM) | 44,7 | 91,1 |
| Cyclosporin A | (0,25 µM) | 69,9 | 77,5 |
| Mycophenolsäure | (0,15 µM) | 89,8 | 76,8 |

2. Kreuzresistenz gegen strukturverwandte, A77 1726 - ähnliche Substanzen. Da keine generelle Resistenz der A20R-Zellen gegen antiproliferative Substanzen vorlag, sollte überprüft werden, ob strukturverwandte Analoga von A77 1726 die gleiche proliferationshemmende Eigenschaft auf A20R-Zellen besitzen, wie auf A20.2J-Zellen. Die Untersuchung wurde mittels Proliferationstest 1 durchgeführt. In der nachfolgenden Tabelle sind vergleichend IC-50-Werte (die Konzentration einer Substanz, welche die Proliferation der Zellen um 50 % hemmt) aufgeführt.

| Testsubstanzen | IC-50 Wert der A20.2J | IC-50-Wert der A20R |
|---|---|---|
| A77 1726 | 2-3 µM | 130 µM |
| X92 0715 | 8 µM | 120 µM |
| X91 0279 | 10 µM | 120 µM |
| X91 0325 | 10 µM | 75 µM |

A20R-Zellen zeigen eine graduell abnehmende Kreuzresistenz zu strukturverwandten A77 1726B-Analoga, was auf eine strukturspezifische Resistenz schließen läßt.
3. Kreuzresistenz der A20R-Zellen gegen Brequinar:
Vorrausgegangene Untersuchungen zum Wirkmechanismus von Leflunomid deuteten auf Parallelen zu Brequinar (Dupont-Merck) hin. Aus diesem Grund wurde Brequinar in die Untersuchungen zur Kreuzresistenz der A20R mit einbezogen.

Ermittelt wurden die IC-50-Werte der A20.2J und A20R-Zellen gegenüber des Brequinar-Natriumsalzes mit Hilfe des Proliferationstestes 1.

| | IC-50-Wert der A20.2J | IC-50-Wert der A20R |
|---|---|---|
| Brequinar-Na⁺-Salz | 0,2 µM | 50-75 µM |

Zusammenfassend läßt sich sagen, daß A20R-Zellen bezüglich ihres Wachstumsverhaltens eine Kreuzresistenz gegen Analoga von A77 1726 und Brequinar, einer Substanz, welche die DHODH hemmt, zeigen.

### Beispiel 3: Untersuchung von A20R-Zellen auf MDR-Proteine

Gelelektrophoretische Auftrennungen der zellulären Proteine der A20.2J und A20R-Zellen zeigten, daß ein Protein mit einem Molekulargewicht von ca. 135 kDa (ermittelt mit Proteineichmarkern) in der resistenten Linie überexpremiert wurde (siehe auch Figur 1). Die erste Vermutung war, daß es sich dabei um ein MDR (Multi-Drug-Resistance) Phänomen handelt.

MDR (Multi-Drug-Resistance) ist definiert als eine Resistenz der Zellen gegen strukturell nicht verwandte antineoplastische Substanzen. Tumorzellen reagieren durch Überexpression eines Plasmamembranglycoproteins, das ATP-abhängig zelltoxische Substanzen aus der Zelle herauspumpen kann. Durch Überexpression dieser MDR-Proteine (135-180 KD) überleben die Zellen auch in höheren Konzentrationen von antiproliferativen Substanzen.

MDR-Proteine können durch Calciumkanalblocker in ihrer Funktion als Ausschleusungspumpen gehemmt werden, was zu einer Anhäufung der Substanz in der Zelle führt. Zu beiden Zellinien wurden deshalb literaturbekannte Calciumkanalblocker und ebenfalls MDR-assozüerte Substanzen zugegeben um zu überprüfen, ob die resistente Linie MDR-Proteine überexpremiert. Als Calciumkanalblocker wurde Verapamil, als MDR-Substrate Daunorubicin und Doxorubicin verwendet. Die Ergebnisse sind unten tabellarisch als % Hemmung der Proliferation dargestellt und wurden mit Hilfe von Test 2 ermittelt.

| Zugabe von 300 nM Daunorucibin | | | Zugabe von 300 nM Doxorubicin | |
|---|---|---|---|---|
| Verapamil (nM) | A.20.2 J | A20R | A20.2 J | A20R |
| 0 | 10.7% | 6,8 % | 2,7 % | 9,1 % |
| 100 | 33,4% | 20,7% | 19,9% | 24,9% |
| 200 | 49,6 % | 31,7 % | 30,4 % | 48,7% |
| 400 | 54,0 % | 42,4% | 40,4% | 47,3% |

Beide Zellinien werden durch die beiden Substanzen in gleichem Maße gehemmt. Die resistenzen A20R-Zellen zeigen keine höhere Akzeptanz durch erhöhte MDR-Expression.

Der gleiche Versuchsansatz wurde gewählt um zu überprüfen, ob A77 1726 ein MDR-transportiertes Molekül ist.

| Verapamil (nM) | A20.2J + 1,6 µM A77 1726 | A20R + 62,5 µM A77 1726 |
|---|---|---|
| 0 | 16.4% | 10.3% |
| 100 | 14,3% | 6,4% |
| 200 | 12,5% | 9,9% |
| 400 | 7,9 % | 13,9% |

Bei den Zellinien konnte festgestellt werden, daß A77 1726 nicht von MDR-Proteinen transportiert wird.

### Beispiel 4: Mikropräparative Reinigung eines 135 kD Proteins

### a.) Probenvorbereitung für Proteinbestimmung

a.1) Proteinbestimmung nach Popov

### (Popov et al.: Acta Biol. Med. Germ. 34, S.1441-1461)

Prinzip: Verdünnte Proteinlösungen werden mit Amidoschwarz/Methanoi/Essigsäure als gefärbtes Pellet ausgefällt, gewaschen, in 0,1 M NaOH aufgenommen und die Extinktion bei 620 nm gemessen.

Die Berechnung des Proteingehalts erfolgt anhand einer Eichkurve mit BSA-Lösungen (BSA = Rinder-Serum-Albumin).

Anmerkung: Diese Proteinbestimmung wird nicht beeinflußt von Detergenzien (SDS, Nonidet, etc.), ebenso stört die Anwesenheit von β -Mercaptoethanol nicht. Es sollten Original Eppendorf®-Gefäße benutzt werden, da die Haftung des Pellets an der Kunststoffoberfläche stark ist und somit Protein-Verluste durch Ablösen des Pellets beim Abgießen der Waschlösungen vermieden werden.

Folgende Lösungen werden benötigt:
"Lösung Popov 1":
   0,65 g Amidoschwarz
   50 ml Popov 2 mind. 1 h rühren, nur eine Woche haltbar.
"Lösung Popov 2":
   50 ml Eisessig
   450 ml Methanol
"Lösung Popov 3":
   4 ml Popov 1
   36 ml Popov 2, anschl. filtrieren

### Erstellung der Eichkurve:

Ansetzen der BSA- Lösung: Rinder-Albumin, v. Sigma, 98 bis 99%ig wird in einer Konzentration von 1 mg/ml in 5%iger SDS-Lösung angesetzt. Es wird eine größere Menge Lösung hergestellt, die in 1 ml-Portionen bei -25°C gelagert wird. Eine 1 ml-Portion wird aufgetaut und anschließend 10 Minuten bei 95°C im Thermomixer (Eppendorf Thermomixer 5436) kräftig geschüttelt. Nach dem Abkühlen werden folgende Verdünnungen vorgenommen:

| 10 µl BSA-Lösg. | 990 µl 5 %ige SDS-Lösg. | 0,010 mg BSA/ml |
|---|---|---|
| 25 " | 975 " | 0,025 " |
| 50 " | 950 " | 0,050 " |
| 75 " | 925 " | 0,075 " |
| 100 " | 900 " | 0,100 " |
| 150 " | 850 " | 0,150 " |
| 200 " | 800 " | 0,200 " |
| ohne " | 1000 " | Blindwert |

Von allen 8 Lösungen werden jeweils zweimal 200 µl (Doppelbestimmung) entnommen, mit 600 µl "Popov 3" versetzt, anschließend kurz und kräftig vermischt (Vortex).

Dann: 5 Minuten zentrifugieren mit 14000 rpm in einer Tischzentrifuge (Eppendorf), der Überstand wird verworfen. Anschließend wird das Pellet 3mal mit je 750 µl "Popov 2" gewaschen und abzentrifugiert. Nach dem letzten Waschgang wird das Pellet in 1 ml 0,1 M NaOH aufgenommen und in einer Kunststoffküvette (d = 1 cm) gegen den Blindwert bei 620 nm die Extinktion gemessen (Spektralphotometer der Fa. Kontron).

### Beispiel für eine Meßreihe:

| Konzentration BSA (mg/ml) | Extinktion bei 620 nm |
|---|---|
| 0 | 0 |
| 0,010 | 0,0459 |
| 0,025 | 0,1154 |
| 0,050 | 0,2442 |
| 0,075 | 0,4025 |
| 0,100 | 0,4964 |
| 0,150 | 0,6856 |
| 0,200 | 0,9534 |

Der Korrelationskoeffizient bei der Auswertung: Proteinkonzentration/Extinktion beträgt erfahrungsgemäß 0,995-0,999 (bei diesem Beispiel bei 0,998)

### a.2) Probeovorbereitung / Proteinbestimmung der A 20-Zellen:

10⁷ A20-Zellen (der Begriff A20-Zellen bedeutet A20.2J und A20R), vorliegend in 1 ml PBS-Puffer, werden innerhalb 5 bis 10 Sekunden mit 10⁴ rpm in der Tischzentrifuge (Eppendorf-Modell 5415 C) zentrifugiert. Der Überstand wird verworfen, das Pellet wird mit 1 ml 5 % SDS-Lösung versetzt, mit einer Pipette mehrmals aufgesaugt und damit homogenisiert und 10 Minuten bei ca. 95°C im Thermomixer kräftig geschüttelt und anschließend abgekühlt.

Von dieser Lösung werden:
20 µl mit 980 µl 5% SDS-Lösung - 50fache Verdünnung
sowie 50 µl mit 950 µl " " → 20fache " versetzt und 10 Minuten bei 95°C im Thermomixer kräftig geschüttelt und abgekühlt. Anschließend werden von jeder Lösung für Doppelbestimmungen zweimal 200 µl entnommen, mit 600 µl "Popov 3" versetzt und so wie oben für BSA beschrieben weiterbehandelt. Die Auswertung erfolgt anhand der bereits beschriebenen Eichkurve.

### Erhaltene Meßwerte:

| Verdünnung | Extinktion bei 620nm | →Proteinkonz. × Verdünnungsfaktor (mg/ml) |
|---|---|---|
| 50fach | 0,0972 | 0,915 |
| 20fach | 0,1800 | 0,720 |

Ergebnis: A20-Zellen enthalten ca. 800 µg Protein.

### b.) Probenvorbereitung für SDS-PAGE

10⁷ A20-Zellen, vorliegend in 1 ml PBS-Puffer, werden innerhalb 5 bis 10 Sekunden mit 10⁴ rpm in der Tischzentrifuge (Eppendorf-Modell 5415 C) zentrifugiert.

### b.1) Direkte Lyse

Der Überstand wird verworfen, das Pellet wird mit 400 µl Probenpuffer versetzt und durch mehrfaches Ansaugen mit der Pipette homogenisiert, kräftig geschüttelt (Vortex-Schüttler) und für 5 bis 10 Minuten bei 95°C im o.g. Thermoschüttler oder Wasserbad bewegt. Die Proteinkonzentration dieser hochviskosen Lösung beträgt ca. 2 mg/ml. Für ein Coomassie-gefärbtes Gel werden von dieser Lösung 40 bis 50 µl/Probentasche, entsprechend 80 bis 100 µg Protein, benötigt. Für Ag-gefärbte Gele wird die beschriebene Lösung anschließend noch 1 : 20 verdünnt, 40 bis 50 µl entsprechen damit einer Proteinkonzentration von 4 bis 5 µg/Probentasche.

### Zusammensetzung des Probenpuffers:

| | |
|---|---|
| Millipore-H₂O | 2,7 ml |
| Glycerin, 98%ig | 10,0 ml |
| 0,25M Tris / 1M Glycin | 9,0 ml |
| 25% SDS-Lsg. | 6,8 ml |
| 0, 1 % Bromphenolblaulsg. | 2,5 ml |
| 2-Mercaptoethanol | 4,0 ml |

### b.2) Einfrieren der Zellen und anschließende Lyse

Das Zellpellet wird sofort im verschlossenen Eppendorf-Gefäß für ca. 1 Minute in flüssigen Stickstoff getaucht und bei -80°C aufbewahrt. Beim Lysieren wird der Probenpuffer direkt auf das tiefgekühlte Zellpellet gegeben.

### c.) SDS-PAGE

Es wurden verschiedene Polyacrylamid-Gele verwendet (10 %, 12 %, 4 bis 22,5 % PAA). Beste Ergebnisse hinsichtlich Bandenschärfe wurden mit Gradientengelen erhalten, deren PAA-Gehalt bei 4 bis 10 % lag. Die hierfür benötigten Techniken/Lösungen werden nachfolgend beschrieben:

### Trenngel

Zusammensetzung der Gellösungen für Gradientengel 4 bis 10 % AA für ein Gel (ca. 24 ml):

| Komponente | 4 % AA-Lsg. | 10 % AA-Lsg. |
|---|---|---|
| H₂O | 7 ml | - |
| Glycerin | - | 6,1 g |
| Stammlsg.1 | 1,6 ml | 4 ml |
| 3M Tris, pH 8,8 | 3 ml | 3 ml |
| 10% APS | 80 µl | 40 µl |
| 10% SDS | 120 µl | 120 µl |
| TEMED | 10 µl | 10 µl |

Stammlösung 1: 30 % Acrylamid / 0,5 % N,N'Methylenbisacrylamid
crosslinking: 1,7%
APS: Ammoniumpersulfat

### Sammelgel

Zusammensetzung der Gellösung mit 3,8 % AA für zwei Gele (ca. 10,5ml)

| Komponente | |
|---|---|
| H₂O | 3,7 ml |
| Stammlsg.2 | 4,0 ml |
| 0,5M Tris, pH 6,8 | 2,5 ml |
| 10 % APS | 200 µl |
| 10 % SDS | 100 µl |
| TEMED | 12 µl |

Das Gel wird nach bekannten Standardmethoden gegossen und nach ausreichender Polymerisierung in einer Vertikal-Elektrophorese-Kammer fixiert. Für ein Coomassie-/Silber gefärbtes Gel werden von der unter b) beschriebenen A 20-Probe je 40 µl = 80 µg / 4 µg Protein pro Probentasche eingefüllt.

Als Molekulargewichtsstandard dienten "Combithek"-Marker von Boehringer Mannheim, dessen Molekulargewichtsbereich im reduzierenden Probenpuffer von 170 bis 14 kD reicht.

Zusammensetzung des Elektrophorese-Laufpuffers:
gebrauchsfertige Verdünnung mit Milli Q-H₂O

| | |
|---|---|
| SDS | 0,1% |
| Tris | 50 mM |
| Glycin | 200mM |

Strombedingungen: ca.5 Stunden bei 35mA / Gel (Spannung 400V) bei Verwendung eines Gels mit den Maßen 17 x 18 x 0,1 cm.

Färbungen:

### 1. Coomassie-Färbung

| Reihenfolge | Zeit | Zusammensetzung der Lösung |
|---|---|---|
| Fixieren / Färben | 20-30 min | 0,2 % Coomassie Brillant-Blue R 250 in 50 %Methanol / 10 %Essigsäure / 40 %H₂O |
| Entfärben | beliebig, Lsg. mehrfach wechseln | 20 % i-Propanol, 7 % Essigsäure, 3 % Glycerin, 70 % H₂O |

### 2. Ag-Färbung (abgewandelte Heukeskoven-Färbung)

| Reihenfolge | Zeit | Zusammensetzung der Lösung |
|---|---|---|
| Fixieren | 30 min | 40%Ethanol, 10%Essigsre.,50%H₂O |
| Inkubieren | 2-24 h | 0,40 g Natriumthiosulfat*5 H₂O + 5,00 g Natriumacetat + 60 ml Ethanel kurz vor Gebrauch: + 1,0 ml Glutardialdehyd (25%ig) mit H₂O auf 200 ml auffüllen |
| Waschen | 3 x 5-10 min | H₂O |

| Färben | 45 min | 200 mg Silbernitrat kurz vor Gebrauch: + 40 µl Formaldehydlsg., 35%ig mit H₂O auf 200 ml auffüllen |
|---|---|---|
| Waschen | 10 sek | H₂O |
| Entwickeln | 2-10 min | 5 g Natriumkarbonat kurz vor Gebrauch: + 20 µl Formaldehyd, ca. 35%ig mit H₂O auf 200 ml auffüllen |
| Stoppen | 10 min | 1,5%ige Na₂EDTA * 2H₂O |

Alle o.g. Schritte werden in leichter Bewegung (Schütteltisch) in jeweils 200 ml/Gel durchgeführt.

Vor dem Fotografieren/Scannen/Trocknen oder Einschweißen in Plastikbeutel wird das Gel mehrere Stunden bis über Nacht in bidest. H₂O inkubiert.

Aufbewahrung: die eingeschweißten Gele werden bei Raumtemperatur bzw. in einem Kühlschrank (T: >0°C!) übereinandergestapelt aufbewahrt, möglichst lichtgeschützt.

### Auswertung / Beurteilung der Gele

Im hochmolekularen Bereich (zwischen Markerbande 170 und 116 kD) kann eine Proteinbande erkannt werden, die bei resistenten A20-Zellen viel stärker exprimiert wird. Dies kann sowohl bei Coomassie- als auch bei Silberfärbung beobachtet werden (siehe Fig. 1).

### Aussage zum Molekulargewicht

Von den acht Kallibrierstandards wurde die Laufstrecke der einzelnen Proteine im 4 bis 10%-Gel im Verhältnis zum Logarithmus des Molekulargewichts aufgetragen. Von der oben erwähnten Proteinbande mit bekannter Laufstrecke konnte somit das Molekulargewicht errechnet werden. Die gesamte Laufstrecke betrug 11,2 cm.

| Proteinbez der Combithek-Marker | Mᵣ (D)/log Mᵣ | Laufstrecke (cm) | R_{f}-Wert |
|---|---|---|---|
| α₂-Macroglobulin (Pferdeplasma) | 170000/5,230 | 4,37 | 0,39 |
| β-Galactosidase (E.coli) | 116353/5,066 | 5,78 | 0,516 |
| Fructose-6-phosphat-Kinase (Kaninchenmuskel) | 85204/4,930 | 7,20 | 0,643 |
| Glutamat-Dehydrogenase (Rinderleber) | 55562/4,745 | 8,35 | 0,746 |
| Aldolase (Kaninchenmuskel) | 39212/4,593 | 9,17 | 0,819 |
| Triosephosphat-Isomerase (Kaninchenmuskel) | 26626/4,425 | 10,00 | 0,893 |
| Trypsin-Inhibitor (Sojabohnen) | 20100/4,303 | 10,33 | 0,922 |
| Lysozym (Hühnereiweiß) | 14307/4,156 | 10,63 | 0,949 |
| unbekanntes Protein, 5 Auftragungen | ? | 5,58-5,65 (5,60) | 0,500 |

Der Mittelwert der 5 Auftragswerte des unbekannten Proteins ist in Klammern angegeben. Der Korrelationskoeffizient betrug 0,977. Das errechnete Molekulargewicht beträgt Mᵣ 135 kDa.

### Densitometrische Auswertung zur Mengenangabe

Am Bio Image® -System (Fa. Millipore, Eschborn) wurde im "whole band-menu" eine Quantifizierung der Banden eines Coomassie-gefärbten PAA-Gels (4 bis 10 %) mit resistenten A 20-Zellen (A20R) vorgenommen. Ergebnis bei 5 ausgewerteten Bahnen mit unterschiedlichem Proteingehalt:

| Gesamtproteinmenge (µg) | IOD = integrierte optische Dichte, (%) der 135kD Proteinbande |
|---|---|
| 80 | 1,07 |
| 80 | 1,03 |
| 60 | 1,05 |
| 60 | 1,04 |
| 40 | 1,32 |

Demnach beträgt der Anteil des 135 kD-Proteins in resistenten A20-Zellen ca. 1 %. Bei normalen A20-Zellen (A20.2 J) konnte bei Auftragung von 80 µg Protein diese Bande nicht quantitativ erfaßt werden, da sie im Vergleich zu den resistenten Zellen nur sehr schwach erkennbar ist.

Sonstige Informationen, die mit der SDS-PAGE erhalten werden: Bei einer Probenaufarbeitung wurde der unter a) beschriebene Probenpuffer verändert, indem kein Mercaptoethanol zugesetzt wurde. Unter dieser Bedingung spalten sich die Proteine nicht in Untereinheiten auf, die durch S-S-Brücken gebildet werden.
Ergebnis: Es kam zu keiner Veränderung des Molekulargewichtes des 135kD-Proteins.

### d.) Mikropräparative Anreicherung des 135 kDa-Proteins

Die zur Sequenzierung benötigte Proteinmenge wird im allgemeinen mit 100 pMol angegeben, das entspricht ca. 14 µg Protein. Bei vorsichtiger Schätzung (im Vergleich zur Konzentration der Marker) wurde die Konzentration des 135kDa-Proteins bei einer 80 µg-Gesamtauftragung
auf 0,3 µg geschätzt. Es wurden 16 Gele (PAA 4 bis 10 %) mit insgesamt 104 der 135 kDa Banden hergestellt.
Die aufgetragene Gesamtproteinmenge betrug stets 80 µg.

### e.) Proteaseverdau im Polyacrylamidgel

Nach SDS-PAGE und Coomassie-Färbung wurden die Gelbanden ausgeschnitten und innerhalb eines Tages durch mehrmaliges Wechseln des H₂O neutralgewaschen. Die Gelstücke wurden anschließend durch ein 32 µm-Sieb (in einer Spritze ohne Kanüle) gedrückt. Die feine Gelpaste wurde anschließend in einer Vakuumzentrifuge bis fast zur Trockene eingedampft.

Danach erfolgte die Zugabe von Enzym/Puffer; es wurde Endoproteinase LYS-C (Boehringer Mannheim) in 10fachem Überschuß zugegeben. Es wurde 6 bis 7 Stunden bei 37°C inkubiert, anschließend für mehrere Stunden mit 1 ml: 60 % Acetonitril / 0,1% TFA, bei 37°C eluiert. Der Überstand wurde abpipettiert und die Elution über Nacht bei Raumtemperatur wiederholt. Der Überstand wurde nun abpipettiert, mit dem ersten Überstand vereinigt, durch einen 0,02 µm Filter (Anatop® von Merck) wiederholt filtriert und in einer Vakuumzentrifuge eingedampft.

Vor dem Einspritzen in die HPLC wird mit 10-20% Ameisensäure verdünnt.

### f.) Peptidtrennung in der HPLC

### Meßbedingungen:

| | | |
|---|---|---|
| Säule : | Superspher® 60 RP Select B | |
| Laufmittel A : | 0,1 % TFA (Trifluoressigsäure) in H₂O | |
| Laufmittel B : | 0,1 % TFA in Acetonitril | |
| Gradient : | t [min] | % B |
| | 0 | 0 |
| | 60 | 60 |
| | 65 | 70 |
| Fluß : | 0,3 ml/min | |
| Meßwellenlänge : | 206 nm | |

Das Ergebnis ist in Figur 2 gezeigt.

### g.) Automatische N-terminale Proteinsequenzanalyse nach Edmanr (Beckmann Analyzer)

| | |
|---|---|
| Peptid 1 | KLG DI MGVK KE (SEQ ID NO: 1) |
| Peptid 2 | KLG DI MGVK KETEPDK (SEQ ID NO: 2) |
| Peptid 3 | KLIVTSATMDA E K (SEQ ID NO:3) |
| Peptid 4 | DATSDLAIIARK (SEQ ID NO:4) |
| Peptid 5 | KIFQ K (SEQ ID NO:5) |
| Peptid 6 | TP O EDYV E AAV (SEQ ID NO:6) |

Die den Peptiden 1 bis 6 entsprechenden Peaks sind in Figur 2 markiert.

### h.) Datenbankvergleich mit bekannten Proteinsequenzen

Die erhaltenen Peptidsequenzen zeigten teilweise starke Homologie zu einem von der Gensequenz abgeleiteten Protein aus Caenorhabtitis elegans, dessen Funktion unbekannt ist. Die mit dieser Sequenz nicht übereinstimmenden Aminosäuren sind markiert (siehe Abschnitt g.). Die außerordentlich starke Homologie von Peptid 3 (aus der Literatur ist bekannt, daß diese SAT-Box in DEAD-Box-Proteinen von Bakterien bis Säugern stark konserviert ist) mit der C. elegans-Sequenz und die fehlende bzw. schwache Übereinstimmung mit Peptid 4 bzw. Peptid 2 sind ein klarer Beweis dafür, daß das erfindungsgemäße Protein ein neuer Vertreter aus der Klasse der DEAD-Box Proteine ist.

Die folgenden Beispiele beschreiben durchgeführte molekularbiologische Experimente. Dabei werden grundlegende molekularbiologische Standardmethoden, die z.B. in "Molecular Cloning - A Laboratory Manual", 2. Auflage von Sambrook et al., erschienen bei Cold Spring Harbor Laboratory Press, beschrieben sind, als bekannt vorausgesetzt. Solche Techniken sind z.B. Präparation von Plasmid-DNA, Plasmid-Minipräparation, Plasmid-Maxipräparation, Elution von DNA-Fragmenten aus Agarosegelen, Elution durch Filtration, Elution durch Adsorption, Enzymatische Modifikation von DNA, Verdau der DNA durch Restriktionsendonukleasen, Transformation von E. coli, Präparation von RNA, RNA-Präparation mit der Einzelschritt-Methode (nach Chomzynski), mRNA-Präparation mit Dynabeads®, RNA-Gele!ektrophorese, Northern-Blot, Radioaktive Markierung von DNA, "Random primed" DNA-Markierung mit [α-32P]dATP, Sequenzierung von DNA nach der Didesoxymethode, cDNA-Herstellung aus Gesamt-RNA, Nicht-radioaktive Markierung von Nukleinsäure, "Random primed" DNA-Markierung mit Digoxigenin (DIG), Nachweis der DIG-markierten Nukleinsäuren.

### Beispiel 5

### PCR-Anreicherung eines cDNA Fragments entsprechend den gefundenen Aminosäuresequenzen.

Die Reaktionen wurden in einem Perkin Elmer Cycler durchgeführt. Für einen 50 µl PCR-Standardansatz wurden folgende Komponenten auf Eis zusammenpipettiert und mit 50 µl Mineralöl überschichtet:
1 µl Template-DNS (0,5-2,5 ng)
1 µl Vorwärtsprimer (30 pmol/µl)
1 µl Rückwärtsprimer (30 pmol/µl)
5 µl dNTP-Gemisch (2 mM je Nucleotid)
5 µl 10x PCR-Puffer
36.5 µl H₂O
0.5 µl Taq-Polymerase (2.5 Units)

Die Amplifikation erfolgte in 40 Zyklen unter folgenden Bedingungen:
1. Schritt: Denaturierung der DNA-Doppelstränge bei 94°C, 30 s.
2. Schritt: Anlagern der Primer an die DNA-Einzelstränge bei 50°C, 2 min.
3. Schritt: DNA-Synthese bei 72°C, 3 min.

Beim letzten Zyklus wurde die DNA-Synthese 5 min lang durchgeführt und der Ansatz danach auf 4°C heruntergekühlt. Für die Analyse wurden 10 µl des Ansatzes auf einem 1 bis 2 %igen Agarosegel analysiert.
Vorwärtsprimer: A20-2, A20-3, A20-4, A20-5 (siehe Tabelle 1)
Rückwärtsprimer: A20-6a, A20-6b (siehe Tabelle 1)
Matrize: A20R-Gesamt-RNA

Vorwärts- und Rückwärtsprimer wurden jeweils paarweise in PCR-Reaktionen kombiniert. Der Ansatz A20-3/A20-6b führte zur Anreicherung einer ca. 630 bp großen cDNA, die zur Überprüfung ihrer Spezifität mit Kombinationen der Primer A20-3, A20-4 und A20-5 mit dem Primer A20-6b reamplifiziert wurde. Zur Erhöhung der Stringenz wurde bei der Reamplifikation eine Anlagerungstemperatur von 55°C gewählt und nur 35 PCR-Zyklen durchgeführt. Nach Klonierung und Sequenzierung des erhaltenen Fragments (Name: A20-5/-6b) mit Standardmethoden erhält man die in Tabelle 2 gezeigten Sequenzdaten.

### Beispiel 6: Northern Hybridisierung

Als Hybridisierungslösung wurde eine fertige ExpressHyb®-Lösung von der Firma Clontech verwendet, die in einer Stunde Hybridisierungszeit die zuvor markierte Gensonde (radioaktiv oder nicht-radioaktiv) an die eventuell vorhandene komplementäre DNA-Sequenz des Trägerfilters bindet.

Zusätzlich benötigte Reagenzien:
20xSSC: 3 M NaCl; 0.3 M Natriumcitrat (pH 7.0)
Waschlösung 1: 2x SSC; 0.05 % SDS
Waschlösung 2: 0.1 x SSC; 0.1 % SDS
Waschlösung 3: 2x SSC; 0.1 % SDS

### 1. Hybridisierung mit nicht radioaktiv markierten Gensonden mit der ExpressHyb® -Lösung (Clontech)

Die ExpressHyb® -Lösung wurde auf 68°C erwärmt und dabei gerührt, so daß keine Präzipitate verblieben. Nun wurde die Membran (10x10-cm) in mindestens 5 ml ExpressHyb® -Lösung prähybridisiert, in dem man bei 68°C für eine halbe Stunde kontinuierlich in einem Hybridisierungsofen mischte. Die nicht radioaktiv markierte DNA-Sonde wurde mit 5 ml frischer ExpressHyb®-Lösung vermengt. Nun ersetzte man die Prähybridisierungslösung durch diese ExpressHyb®-Lösung und ließ den Blot für eine Stunde bei 68°C im Hybridisierungsofen inkubieren. Nach der Inkubation wurde für 30 min mit 20 ml der Waschlösung 3 (pro 100 cm² Membran) bei Raumtemperatur gewaschen, wobei die Lösung einmal ersetzt wurde. Der zweite Waschschritt erfolgte bei 50°C für 30 min mit Waschlösung 2. Auch hier wurde die Lösung einmal ersetzt. Danach ließ man die überschüssige Waschlösung von der Membran abtropfen und konnte die Membran dann direkt für die Chemilumineszenzdetektion verwenden.

### 2. Hybridisierung mit radioaktiv markierten Gensonden mit der ExpressHyb®-Lösung (Clontech)

Die Hybridisierung erfolgte wie bei der nicht-radioaktiv markierten DNA-Sonde. Nach der Inkubation wurde dann aber mit Waschlösung 1 30-40 min bei Raumtemperatur unter mehrmaligen Ersetzen der Lösung gewaschen. Der zweite Waschschritt erfolgte mit Waschlösung 2 für 40 min bei 50°C. Dabei wurde einmal die Lösung ersetzt. Danach ließ man auch hier die überschüssige Waschlösung abtropfen und der Blot wurde in eine Plastikfolie eingeschweißt. Der Blot wurde in einer Exponierungskassette bei -70°C exponiert oder im Phosphoimager (BIORAD) ausgewertet.

Die verwendete RNA und Hybridisierungssonde sind jeweils in den Figurenlegenden angegeben.

### 3. Zeitlicher Verlauf der mRNA-Spiegel der putativen RNA-Helikase unter dem Einfluß von Leflunomid bei A20.J und A20R-Zellen

Das Experiment ist in Figur 3A und 3B und der dazugehörigen Figurenlegende dargestellt. Es zeigt sich bei allen untersuchten Zellen (A20.2J und A20R) eine Bande der Größe 4,4 kb. Die A20R geben ein sehr starkes Signal, A20.2J-Zellen nur ein sehr schwaches Signal, welches aber nach Behandlung dieser Zellen für eine oder 8 Stunden mit A77 1226 etwas stärker wird. A77 1226 induziert die Bildung der hier untersuchten mRNA nicht signifikant.

### 4.) Zeitlicher Verlauf der mRNA-Spiegel der putativen RNA-Helikase bei Absetzung von A77 1226.

Das Experiment ist in Figur 4A und 4B und der dazugehörigen Legende dargestellt. Mit dem Absetzen von A77 1226 sinkt der mRNA-Spiegel der untersuchten mRNA im Beobachtungszeitraum (bis 5 Monate).

### 5.) mRNA Level der putativen RNA-Helikase in acht verschiedenen humanen Geweben.

Das Experiment ist in Figur 5 und der entsprechenden Legende beschrieben. Es zeigt sich, daß die mRNA-Spiegel in den untersuchten Geweben unterschiedlich sind. Da die mRNA-Expression mit der Leflunomidresistenz korreliert (siehe 3), sind muskuläre Organe wie Herz und Skelettmuskel möglicherweise weniger empfindlich gegenüber Leflunomid.

### Beispiel 7: Homologien der putativen murinen RNA-Helikase zu einem humanen cDNA-Klon

Die Aminosäuresequenz KLGDIMGVKK von einem Teilbereich der differentiell exprimierten putativen RNA-Helikase von Leflunomid-resistenten A20R-Zellen wurde in einem Eintrag des cDNA-Klons B 185 (Homo sapiens) in der EM NEW (EMBL-neue Einträge)-Datenbank gefunden. Dadurch war es möglich, passende Primer für die PCR herzustellen, die man in einer PCR mit einer humanen cDNA-Bank als Matrize dazu dienten, eine cDNA korrespondierend zu einer zur entsprechenden humanen putativen RNA-Helikase anzureichern. Die neuen stromaufwärts- und -abwärts-Primer sind in Tabelle 1 als Primer Nr. 7 und 8 wiedergegeben (7 = hs1, 8 = hs2).

Die Bedingungen der PCR wurden stringent gehalten, da die Primer komplementär zur Zielsequenz waren. Die Hybridisierung wurde 45 s bei 55°C durchgeführt, die Denaturierung 30 s bei 94°C und die Synthese nur 45 s bei 72°C. Grund für die kurze Denaturierungs- und Synthesephase war die bekannte Länge des zu erwartenden Inserts (246 bp). Die Konzentrationsverhältnisse der PCR wurden laut Standard gemäß Beispiel 5 gewählt. Als Matrizen dienten drei verschiedene humane cDNA-Banken (hergestellt aus 1. peripheren T-Zellen, 2. PMA-stimulierten HI-60 myeloiden Vorläuferzellen, 3. Placenta). Es wurde jeweils ein 246 bp langes PCR-Fragment erhalten, dessen Sequenz die der Nukleotide 1431 bis 1672 der Tabelle 3 entspricht.

### Beispiel 8: Erhalt des kompletten humanen cDNA Klons kodierend das Gen des Nukleinsäure-bindenden Proteins mit putativen Helikase- und ATPase-Eigenschaften durch Koloniehybridisierung

Aufgrund der Ergebnisse der Northern-Blot-Experimente (Beispiel 6) wurde zum Screening eine aus humanem Skelettmuskel hergestellte cDNA-Bank verwendet. Als Sonde wurde die Sequenz hs1/hs2 eingesetzt. Zur Synthese markierter Sonden-DNA wurde hs1/hs2 DNA über PCR mit den Primern hs1 und hs2 und dem hs1/hs2-Klon (Vektor: pCR™II) als Template amplifiziert und anschließend über Agarosegelelektrophorese und phenolische Elution aufgereinigt. Für die DIG-Markierung mit Hilfe von Zufallsprimern ("random primed labeling") wurden 1 µg hs1 /hs2-DNA als Template eingesetzt und nach 20 h Reaktionszeit ca. 2 µg markierter Sonden-DNA pro 1 µg Template Ausbeute erhalten. Um die Sondenspezifität zu überprüfen wurde eine Verdünnungsreihe von hs1/hs2 DNA von 0,1 pg bis 10 ng auf Nylonmembran fixiert und mit der DIG-markierten hs1/hs2-Sonde hybridisiert. Es zeigte sich, daß 5-25 ng Sonde pro ml Hybridisierungslösung ausreichten um 10 pg hs1/hs2 DNA schwach und ab 100 pg hs 1/hs2 DNA (Hybond N +) deutlich zu detektieren.

Für das erste Screening der Genbank wurden pro 150 mm Agarplatte ca. 40000 Kolonien ausplattiert. insgesamt wurden 20 Masterplatten angefertigt, so daß ca. 800000 Einzelkolonien ausplattiert worden sind. Mit dieser Kolonienzahl schien die Wahrscheinlichkeit ausreichend, daß bei einer vom Hersteller angegebenen Anzahl unabhängiger Klone von 1,1x10⁶ der gesuchte Klon unter den ausplattierten war. Es wurden je 2 also insgesamt 40 Replikafilter angefertigt, die der Hybridisierung mit DIG-Sonde unterzogen wurden. Für diese Hybridisierung wurde eine Sondenkonzentration von 25 ng/ml eingesetzt. Zur Detektion wurden die Membranen für 2 Std. auf Röntgenfilmen exponiert. Es ergaben sich auf 5 verschiedenen Platten insgesamt 19 positive Klone. Von den 19 positiven Klonen aus dem Primärscreening wurden 5 Klone im Sekundärscreening bestätigt. Diese Klone wurden isoliert und charakterisiert. Folgende geschätzte Insertgrößen ergaben sich für die Klone:

| | |
|---|---|
| Klon 1 | 1 ,6 kB |
| Klon 2 | 3,5 kB |
| Klon 3 | 1,6 kB |
| Klon 4 | 0,9 kB |
| Klon 5 | 6,5 kB |

Zwecks weiterer Charakterisierung wurden die Klone ansequenziert und die erhaltenen Teilsequenzen sowie Restriktionskarten miteinander verglichen. Der Vergleich der Sequenzen miteinander bestätigte die Vermutung, daß Klon 1 und Klon 3 fast identisch waren. Es ergab sich, daß die Klone 1 bis 4 einer Gensequenz, die die hs1/hs2-cDNA-Sequenz beinhaltete und einer geschätzten Länge von 4,5 kB entsprach. Mit enthalten schien das vollständige 5'-Ende und der poly-A-Schwanz der mRNA. Die Gesamtlänge ließ vermuten, daß es sich um die vollständige Sequenz handelte, die für die Expression eines 135 kD Proteins erforderlich wäre. Eine schematische Darstellung zeigt die Orientierung der cDNAs zueinander und die Lage der zum Screening verwendeten Sequenz hs1/hs2 (Figur 6A).

Im Vergleich zu den übrigen Klonen schien Klon 5 Unterschiede aufzuweisen. Die Ansequenzierung dieses Klons ergab keinerlei Überlappungen mit den anderen Sequenzen und auch keinen Hinweis auf die Lage der hs1/hs2-Sequenz im Klon. Bereits im Verlauf der Restriktionsanalyse zeigte das Plasmid 5 Besonderheiten, die vermuten ließen, daß es nicht aus dem gleichen Gen stammte wie die übrigen Klone. Auch die außergewöhnliche Länge des Inserts von geschätzten 6,5 kB sprach für eine aufgrund von Hybridisierungsartefakten isolierte cDNA, so daß deren Untersuchung vorläufig zurückgestellt wurde.

Klon 1 und Klon 2 wurden vollständig sequenziert. Die Daten der Sequenzierung sind in Tabelle 3 dargestellt.

Es handelte sich um eine DNA-Sequenz von 4,3 kB Gesamtlänge, wobei Klon 1 genau 1590 und Klon 2 3210 Basenpaare lang waren und in einem Bereich von 530 Basenpaaren überlappten. Die zuvor bekannte Sequenz hs1/hs2 lag zwischen Position 1430 und 1672. Die Lage dieser Sequenz war ein Indiz dafür, daß der erste (mit der ersten Base beginnende) der sechs möglichen Leserahmen der richtige war. In diesem Leseraster lagen zwei Stop-Codons: Eines an Basenposition 58 (TGA) und eines an Position 3729 (TGA), wonach ca. 300 Basenpaare stromabwärts ein poly-A-Schwanz folgte. Nach dem ersten Stop folgte an Position 148 ein Methionincodon, das ein mögliches Startcodon für die Translation zu sein scheint, da es nicht nur das erste ATG-Codon in der Sequenz war, sondern auch Charakteristika einer Kozak-Startsequenz aufwies, nämlich einen Purinrest (G) an Position -3 und ein G an Position +4. Knapp 1000 Basenpaare weiter taucht das nächste ATG-Codon, genauer zwei Methionin-Codons in Folge auf. Das zweite Codon könnte aufgrund der Umgebung - ein A auf -3 und ein G auf +4 - ebenfalls ein Start-Codon sein. Da in 90-95% der Fälle bekannter Wirbeltier-mRNA-Translationsinitiation das erste auftauchende Methionincodon im Leseraster gleichzeitig das Start-Codon ist, wurde dies auch für den vorliegenden Fall angenommen. Ausgehend von dieser Annahme, würde die Sequenz für ein 1227 AS langes Protein kodieren. Bei einem mittleren Gewicht von 110 Dalton pro Aminosäure würde das einem Protein von knapp 135 kD entsprechen. Aufgrund der Größe des Proteins, des ununterbrochenen Leserahmens und des relativ eindeutigen Startcodons handelte es sich bei der Sequenz höchstwahrscheinlich um die komplette cDNA.

Bei der genaueren Betrachtung konnte der Homologiebereich zur murinen Sequenz 05/6b bestimmt werden. Der Vergleich der Sequenz 05/6b aus der murinen Zellinie A20R mit der gefudenen humanen Sequenz ergab eine Abweichung von 15 Aminosäuren von 245, was einer prozentualen Abweichung von ca. 6 % entsprach.

### Beispiel 9: Homologiedomänen in der gefundenen humanen Sequenz und Ähnlichkeiten zu anderen Proteinen

Der Sequenzvergleich mit den Homologiedomänen der Superfamilie II möglicher Helikasen ergab, daß alle konservierte Domänen der DEAH-Protein-Familie in der humanen Sequenz vorhanden waren (Abbildung 6B).

Die erste Domäne - das ATPase A-Motiv beginnt mit der 655sten Aminosäure. In den Domänen weichen nur insgesamt zwei Aminosäuren von der Homologiesequenz ab: Ein Prolin statt einem Threonin in Domäne IV und ein Serin statt einem Isoleucin in Domäne VI. Weiterhin ist der Abstand der ersten Homologiedomäne zum N-Terminus mit 654 Aminosäuren um 150 Reste größer als bei bisher bekannten DEAH-Box Proteinen. Eine weitere geringfügige Abweichung ist der Abstand zwischen Domäne IV und V: Statt 75 bis 80 Aminosäureresten liegen hier nur 74 Reste dazwischen. Ansonsten läßt sich das von der humanen cDNA abgeleitete Protein aufgrund der gezeigten Homologien eindeutig in die Familie der DEAH-Box Proteine einordnen. Desweiteren wurde am N-Terminus der Sequenz eine Aminosäureabfolge identifiziert, die zur "nuclear localisation site" (NLS) des SV 40 T-Antigens starke Homologien aufweist. Diese NLS-Homologie beginnt mit der 69sten Aminosäure und ist 10 Reste lang.

Zur weiteren Charakterisierung der putativen Helikase-Sequenz wurde ein Sequenzvergleich im GCG-Programm mit "genembl", "swissprot" und "pir" auf DNA- und auf Proteinebene durchgeführt.

Die Genbankanalyse ergab Homologien zu einigen bereits bekannten Proteinen der DEAH-Proteinfamilie (Tabelle 4).
Als Protein mit den stärksten Homologien wurde K03H1.2 aus C. elegans identifiziert. Dieses Protein wurde seinerseits aufgrund vorhandener Homologiedomänen als mögliches DEAH-Box Protein klassifiziert (Wilson et,al., 1994, Nature 368: 32-38). Ursprünglich sequenzierte Peptidfragmente des 135 kD Proteins aus A20R-Zellen wiesen ebenfalls Ähnlichkeiten mit der Sequenz aus C. elegans auf. Dieser Befund hatte bereits frühzeitig Hinweise geliefert, daß es sich bei dem überexprimierten Protein in A20R um eine mögliche RNA-Helikase handeln könnte. Desweiteren wurde ein auf DNA-Ebene zu 60% homologes Protein identifiziert, das 1994 aus HeLa-Zellen kloniert wurde und als HRH1 bezeichnet wurde (Ono et al., 1994, Molecular and Cellular Biology. 14: 7611-7620) - ebenfalls eine mögliche humane RNA Helikase. Weitere Homologien von ca. 50% auf Proteinebene wurden zu den Spleiß-Faktoren PRP 2, 16 und 22 aus S. cerevisiae festgestellt, ebenfalls Mitglieder der DEAH-Familie (Chen und Lin, Nucl. Acids Res. 18: 6447, 1990; Schwer und Guthrie, Nature 349: 494-499, 1991; Company et al., Nature 349: 487-493, 1991). Ferner wurden signifikante Homologien zu den DEXH-Proteinen MLE aus D. melanogaster (Kuroda et al., 1991, Cell 66: 935-947) und der möglichen nukleären DNA-Helikase II - NDH II - aus dem Rind (42 und 43% auf Proteinebene) gefunden (Zhang et al., 1995, J. Biol. Chem. 270: 16422-16427).

### Beispiel 10: In vitro Expression der humanen putativen RNA-Helikase

Mittels Kaninchen-Retikulozytenlysat wurde eine in vitro Translation der erhaltenen cDNA durchgeführt. Dazu wurden in verschiedenen Ansätzen von linearisierter und zirkulärer DNA zwischen 0,5 und 2,0 µg eingesetzt. Als Positiv-Kontrolle diente die von Promega mitgelieferte Luciferase DNA. Die Translation erfolgte mit T7-Polymerase. Durch Einbau von ³⁵S-Methionin wurde das Genprodukt markiert und konnte so nach Auftrennung auf einem denaturierenden SDS-PAA-Gel im Autoradiogramm sichtbar gemacht werden (nicht gezeigt).

Alle Ansätze ergaben unabhängig von der eingestezten DNA-Menge gute Resultate, wobei die zirkuläre DNA etwas effizienter translatiert wurde als die linearisierte DNA. Die Positiv-Kontrolle zeigte die erwartete Bande der Luciferase bei 61 kD, die Nullkontrolle ohne DNA ergab erwartungsgemäß keine Signale. Bei den Genprodukten der Helikase-cDNA lag die Hauptbande an synthetisiertem Protein mit der eindeutig stärksten Proteinkonzentration zwischen den Proteinstandards für 97,4 und 220 kD. Darunter waren schwächere Banden kleinere Translationsprodukte zu erkennen, die wahrscheinlich durch den frühzeitigen Abbruch von Protein- bzw. mRNA-Synthese entstanden waren. Diese unvollständigen Translationsprodukte sind bei einem Protein dieser Größe zu erwarten.

Ein direkter Vergleich zwischen dem nativen Protein aus A20R-Zellen und dem Genprodukt der in vitro Translation sollte sicherstellen, daß es sich tatsächlich um die komplette cDNA handelte. Dazu wurden auf einem SDS-PAA-Gel parallel Zellysate von A20.2J- und A20R-Zellen sowie das in vitro Translationsprodukt der klonierten cDNA-Sequenz und die Nullkontrolle aufgetragen. Da im 50 µl Ansatz des Retikulozytenlysates Proteinmengen zwischen 150 und 500 ng produziert werden (Angaben von Promega bezüglich Luciferase-Kontrolle) und 1/10 des Ansatzes auf eine Geltasche aufgetragen wurde, reichte die Coomassie-Färbung (ab einem Proteingehalt von 100 ng lassen sich Banden anfärben) zum Nachweis des Genproduktes nicht aus. Deshalb wurde zusätzlich zur Coomassie-Färbung ein Autoradiogramm mit einem Röntgenfilm erstellt. Der Film konnte anschließend auf das getrocknete Gel aufgelegt werden, wodurch ein direkter Vergleich der Proteinbanden möglich war (nicht angezeigt). Auf das 7,5 %ige SDS-Gel (Trenngel: 5%) wurden je 5 µl Retikulozytenlysat mit und ohne Helikase-Genprodukt, 20 µl A20R-Lysat und 23 µl A20.2J-Lysat aufgetragen (Volumina jeweils mit SDS-Probenpuffer auf 30 µl aufgefüllt). Als Marker wurden ein "Rainbow-Marker" und ein Coomassie-Marker verwendet. Es zeigte sich, daß bei etwa 135 kD bei A20R-Zellysaten die Proteinbande des überexprimierten Gens der RNA-Helikase im Coomassie gefärbten Gel erscheint. Das gleiche Gel überlagert mit dem dazugehörigen Autoradiogramm zeigt, daß die Bande des cDNA-Volle-Länge-Klons Genproduktes auf der gleichen Höhe wie das 135 kD Protein in A20R liegt.

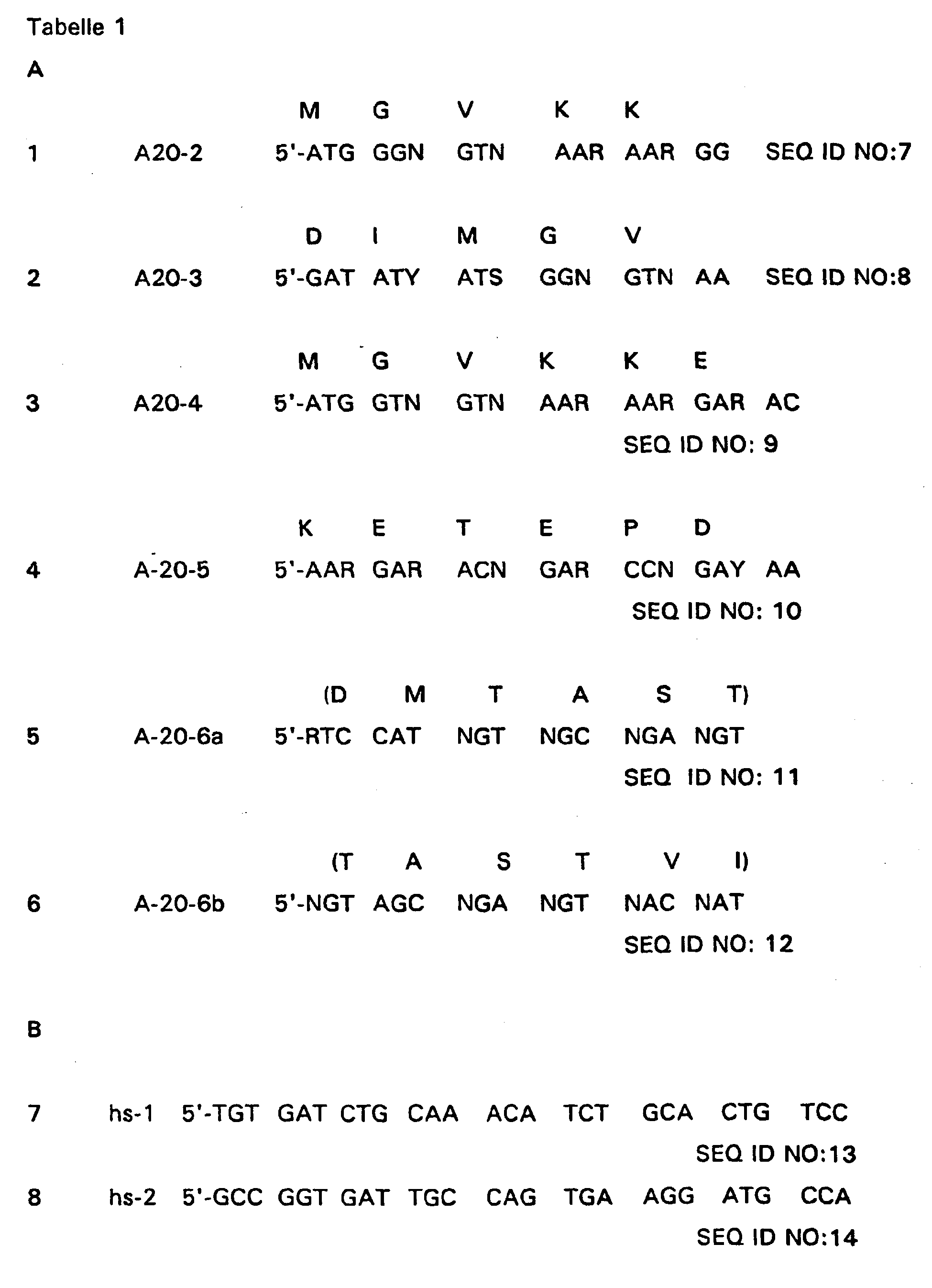

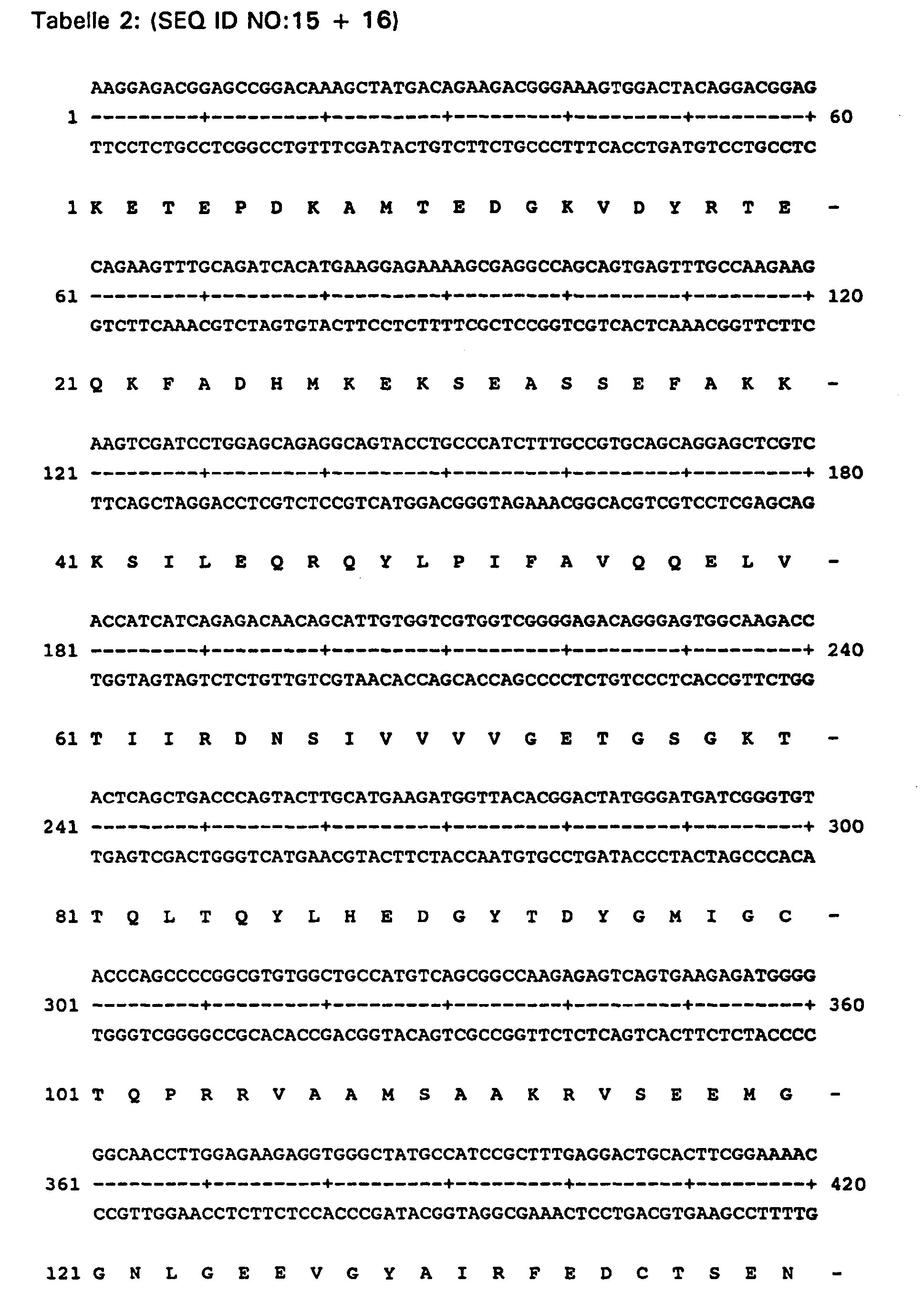

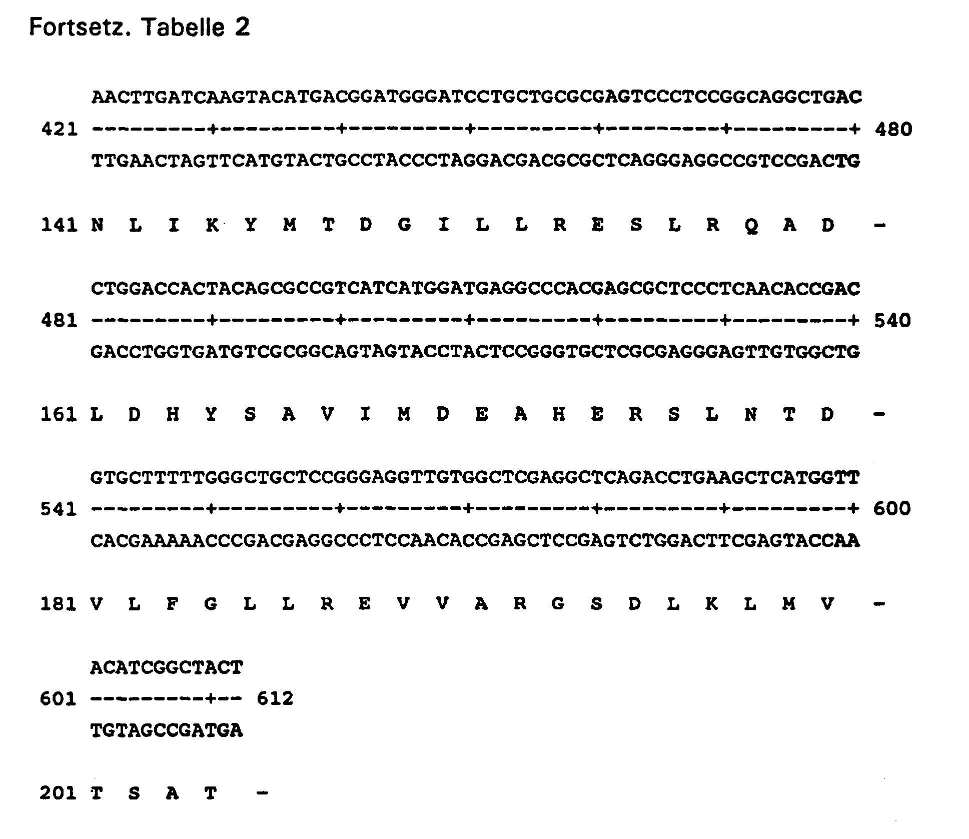

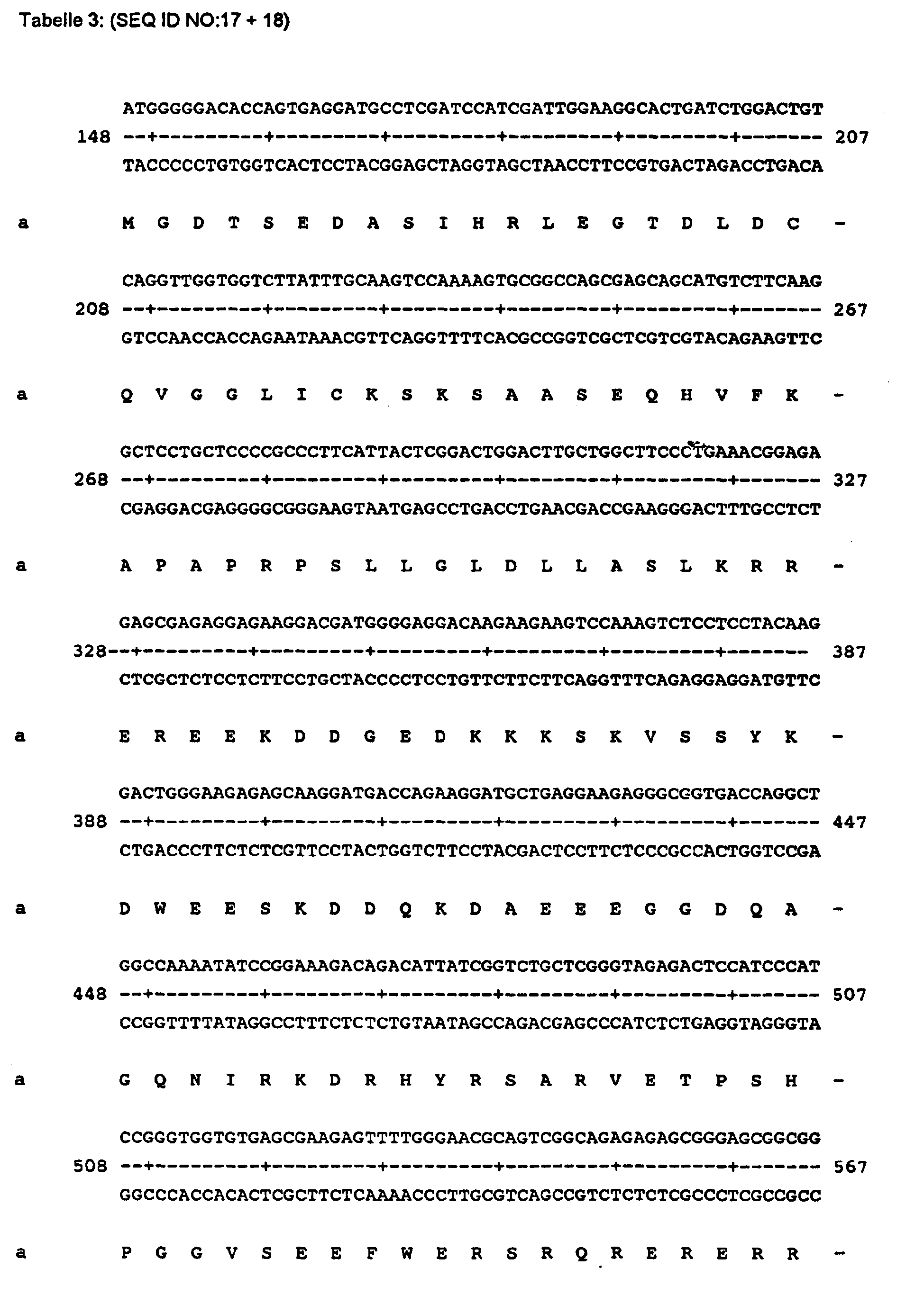

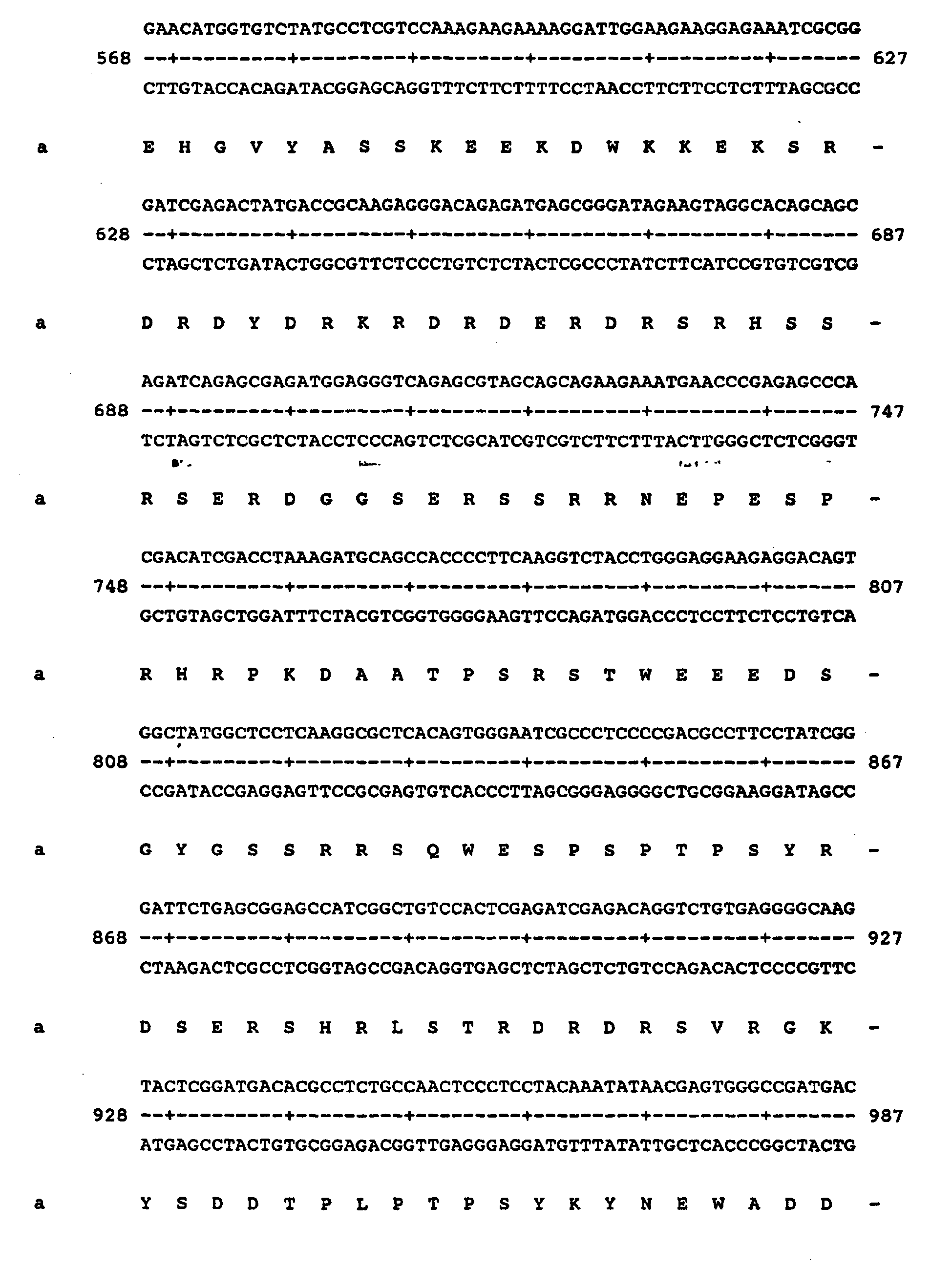

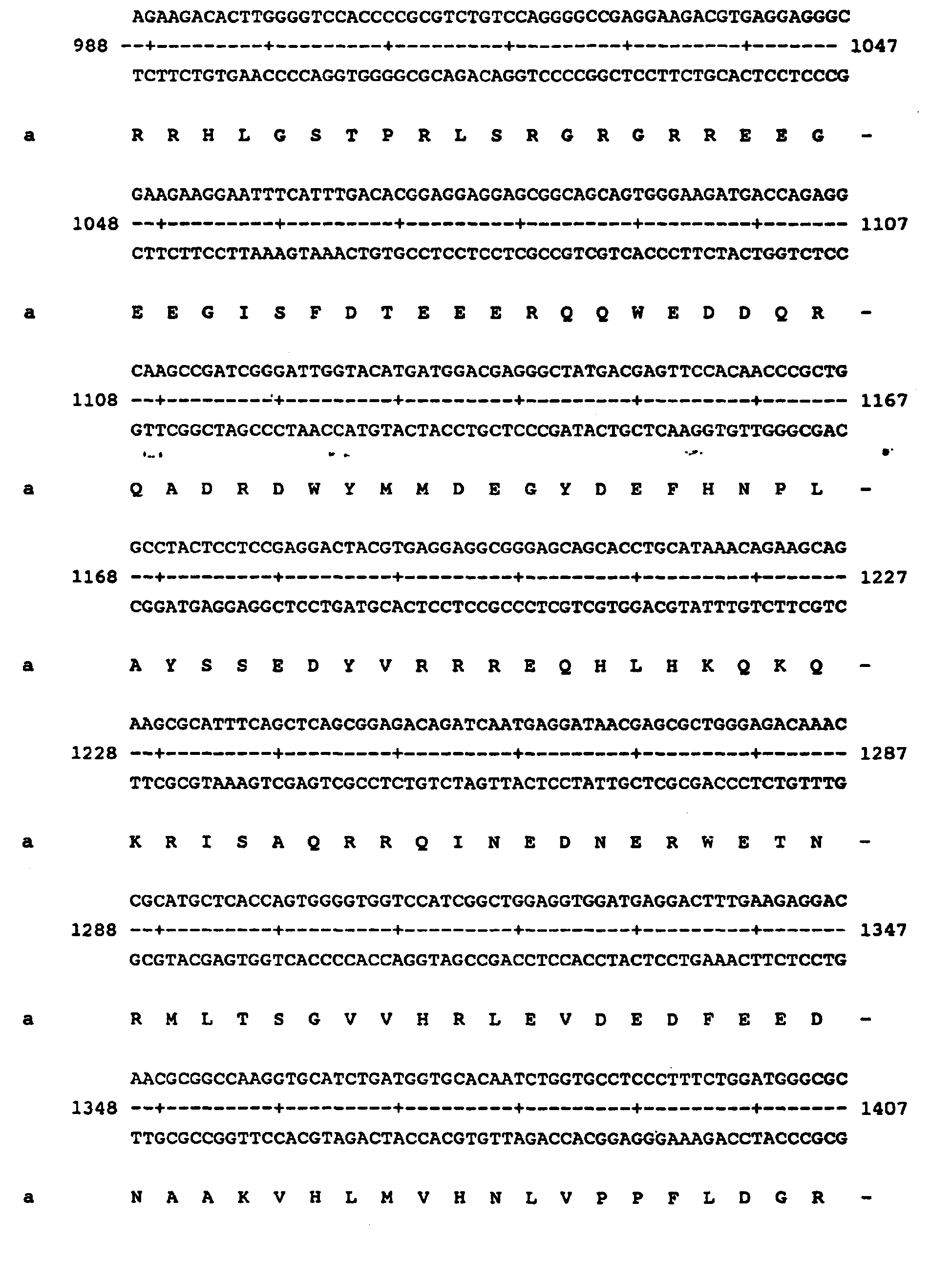

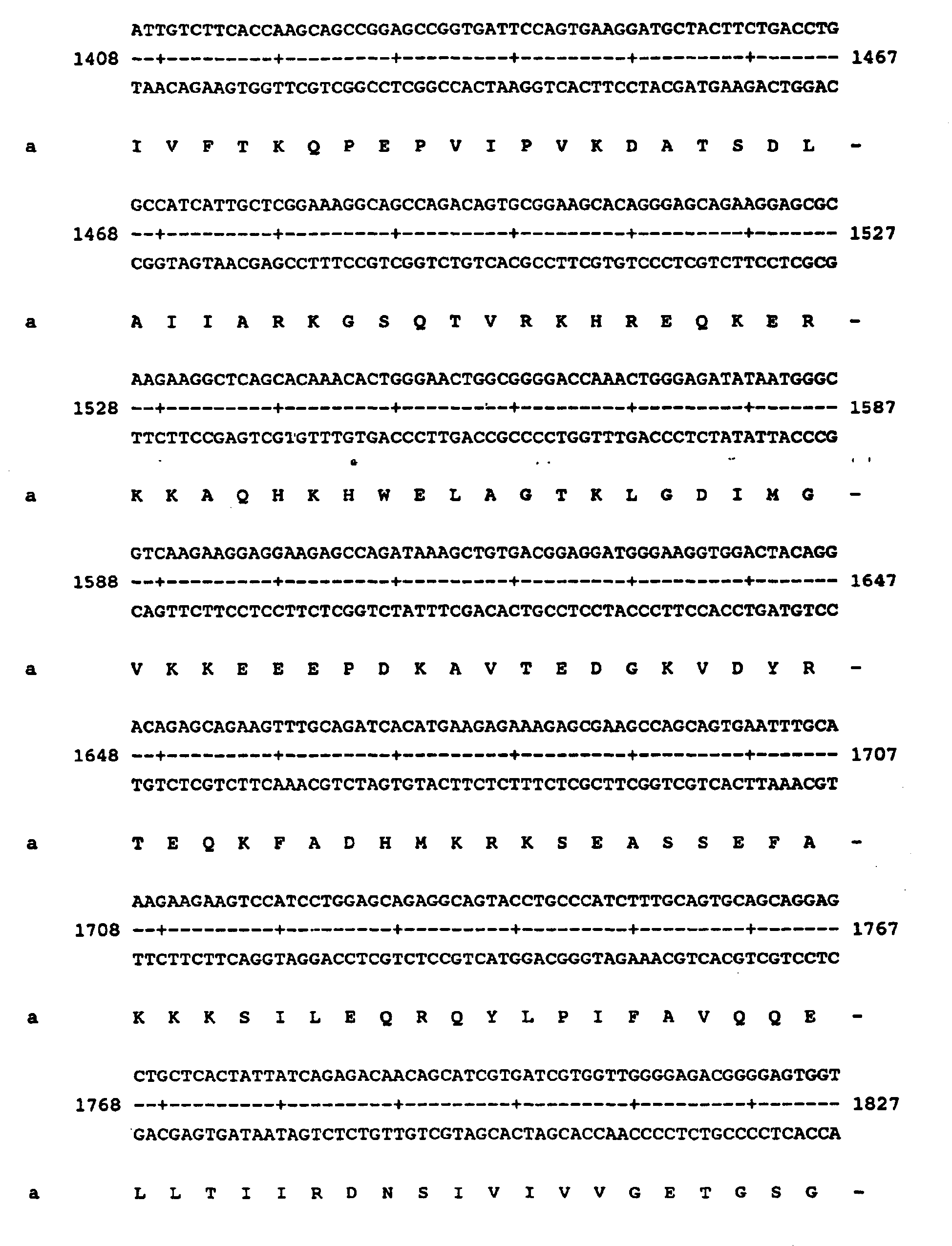

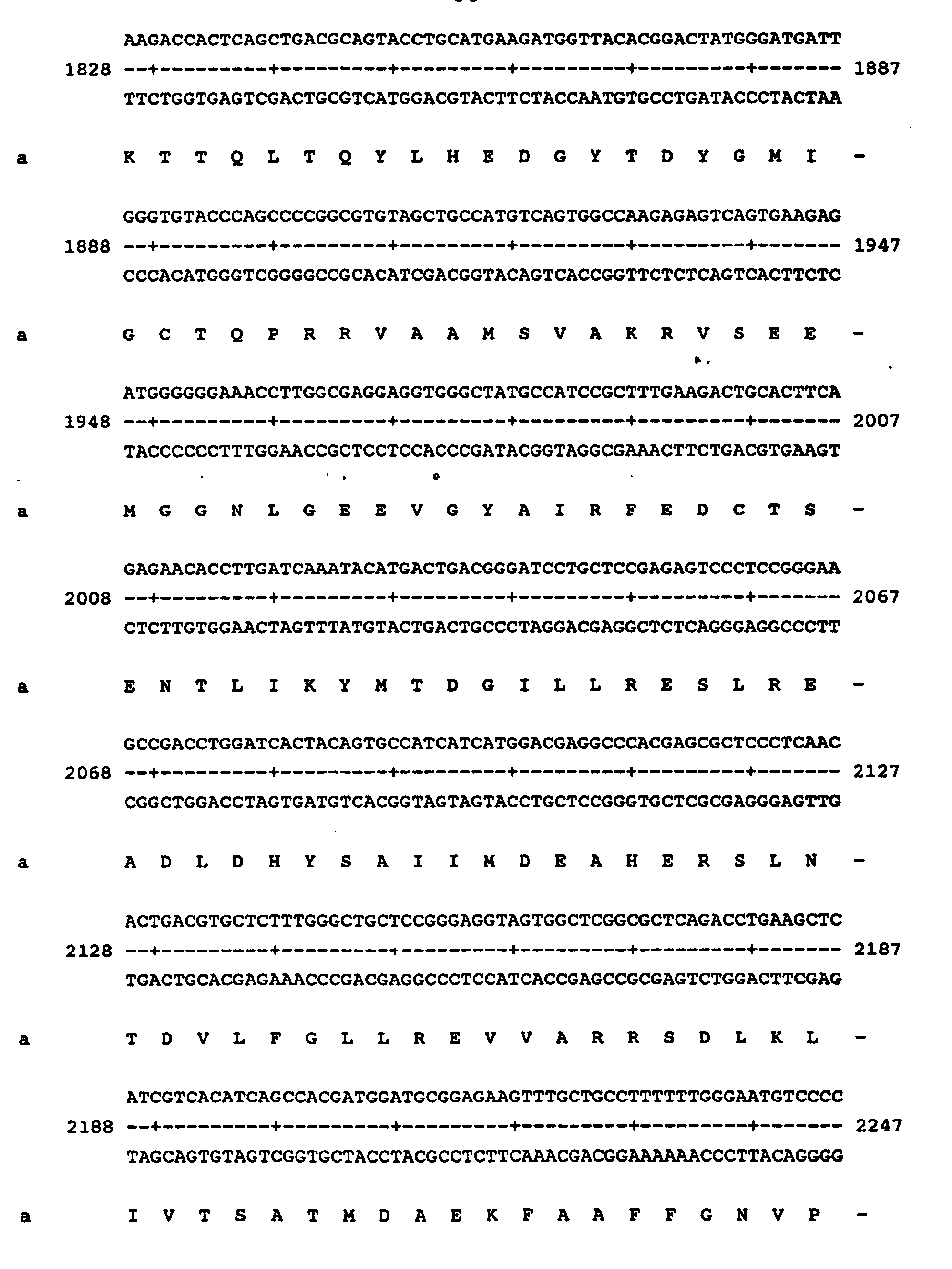

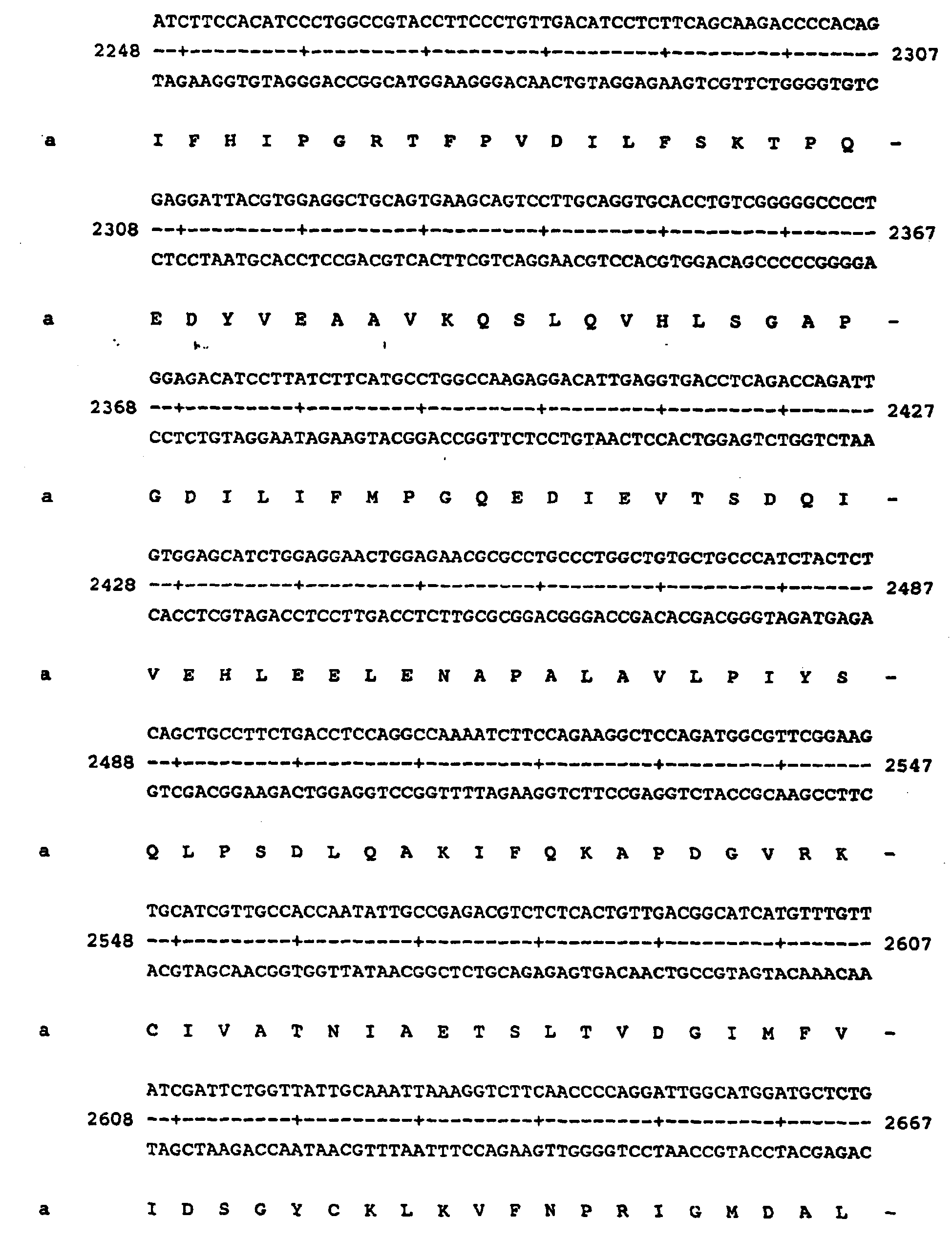

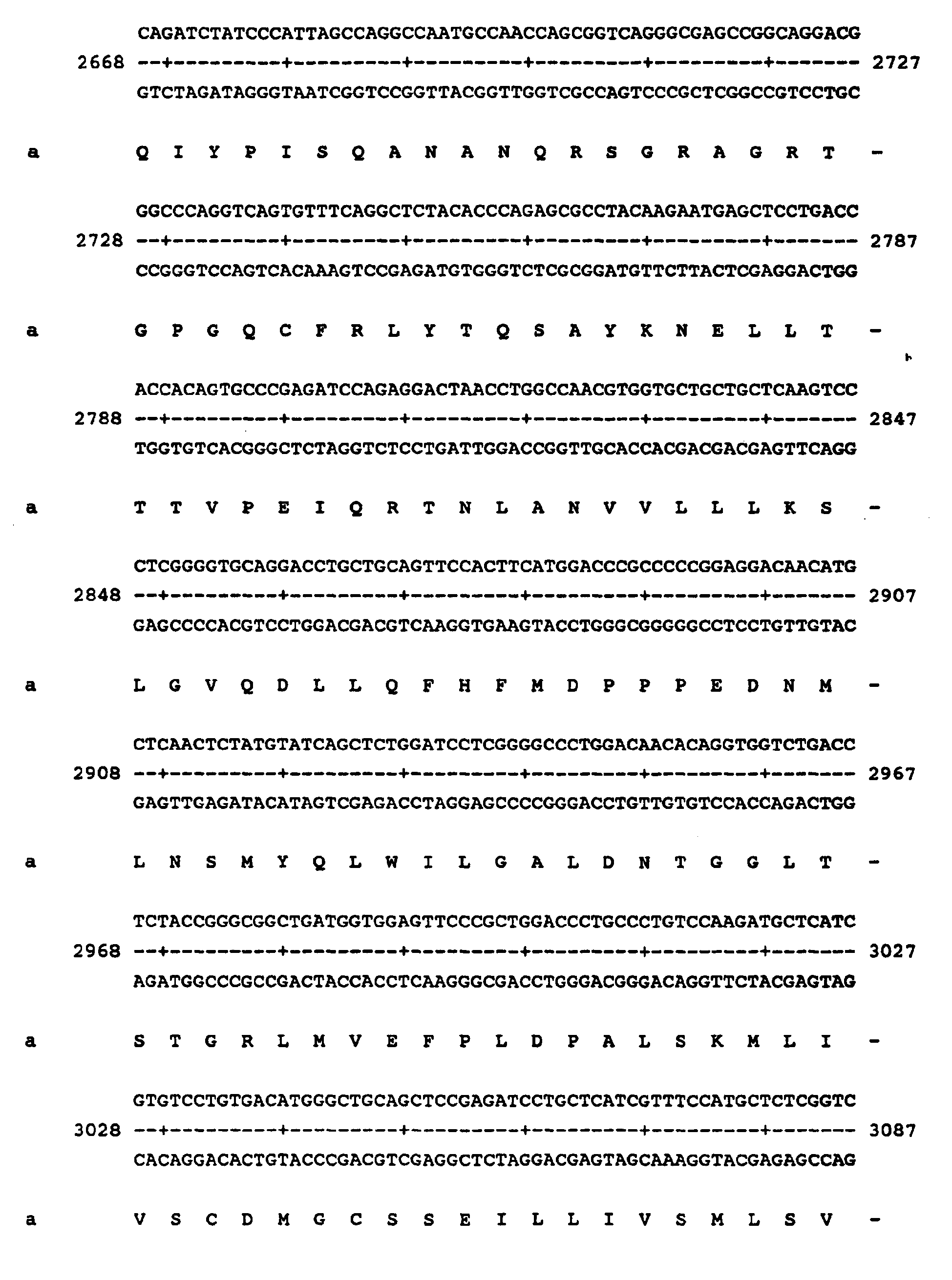

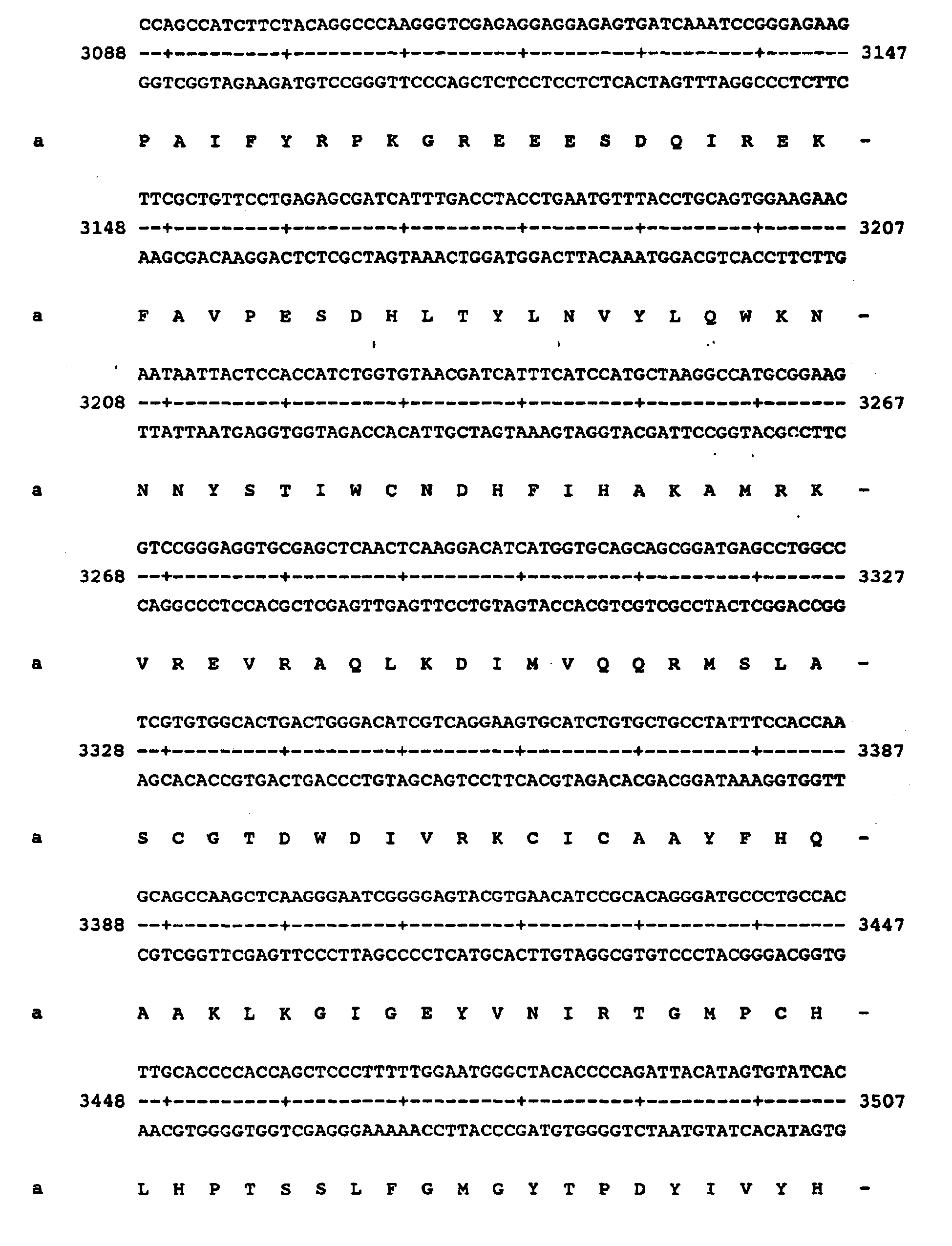

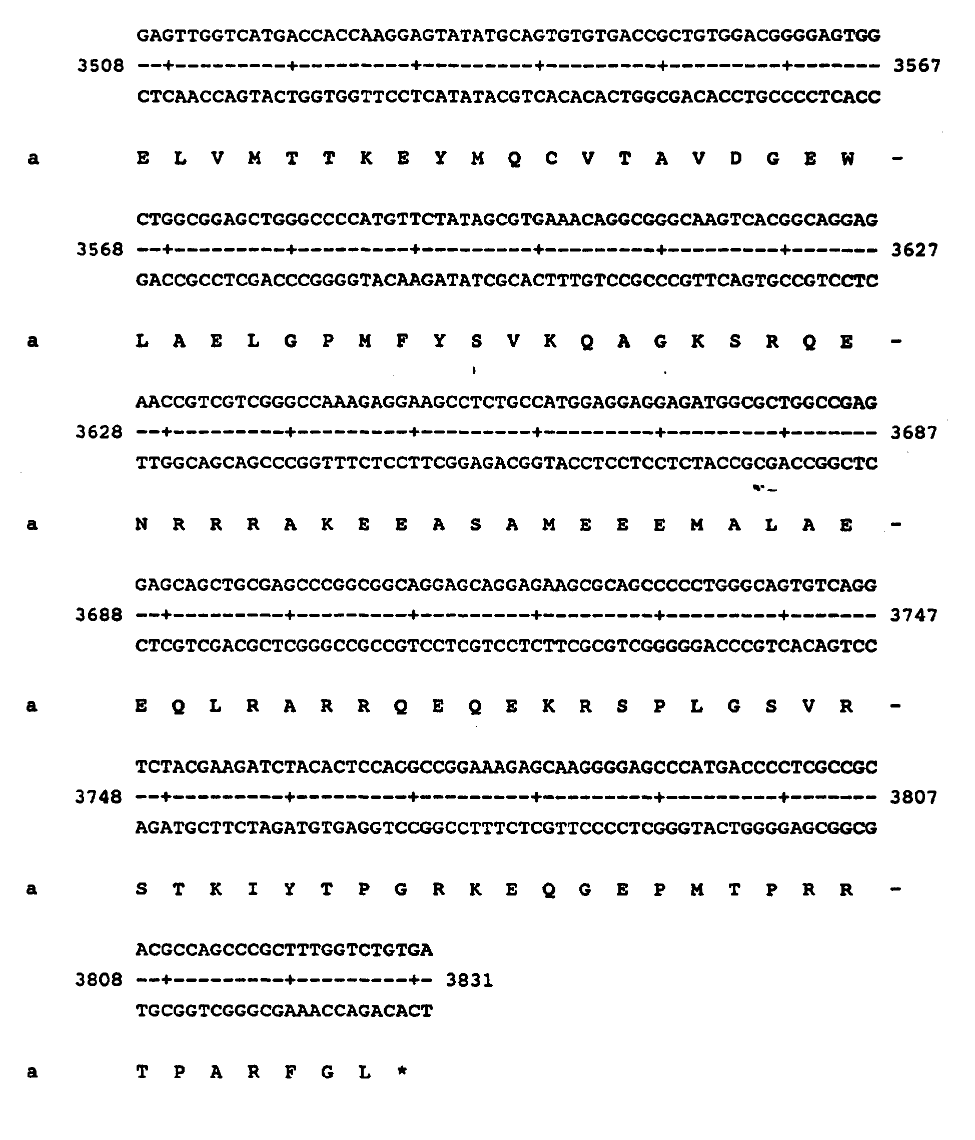

**Tabelle 4:**

| Protein/ Sequenz | Organismus | Übereinstimmmungen | Aufgaben in der Zelle | Biochemische Eigenschaften |
|---|---|---|---|---|
| K03h1.2 | C. elegans | 65 % | mögliche ATP-abhängige RNA-Helikase | ? |
| HRH1 | Mensch | 60 % | humanes Homologes zu PRP 22 | ? |
| PRP16 | S. cerevisiae | 51 % | zweiter Schritt im prä-mRNA-Spleißen; Suppressor von Mutationen im "branch point" | RNA-abhängige ATPase |
| PRP2 | S. cerevisiae | 50 % | erster Schritt im prä-mRNA-Spleißen | RNA-abhängige ATPase |
| PRP22 | S. cerevisiae | 49 % | Freisetzung gespleißter mRNA aus dem Spleißosom | ? |
| MLE | D. melanogaster | 43 % | "dosage compensation"-Ausgleich des fehlenden X-Chromosoms im Männchen | ? |
| NDH II | Rind | 42 % | unbekannt | RNA- und DNA-Helikase-aktivität |

## Patentansprüche

1. Isolierte DNA, enthaltend das Gen für ein Nukleinsäure-bindendes Protein mit Helikase- und ATPase-Eigenschaften, ausgewählt aus der Gruppe bestehend aus:
a) einer DNA enthaltend eine DNA-Sequenz gemäß Tabelle 2;
b) einer DNA enthaltend eine DNA-Sequenz, die in der Lage ist, mit dem DNA-Molekül gemäß (a) unter stringenten Bedingungen zu hybridisieren;
c) einer DNA enthaltend eine DNA-Sequenz, die aufgrund der Degeneriertheit des genetischen Codes von den DNA-Molekülen gemäß (a) oder (b) verschieden ist, jedoch die Expression der entsprechend mit den DNA-Molekülen gemäß (a) und (b) exprimierbaren Proteinen ermöglicht;
d) Teile von DNA-Molekülen gemäß (a), (b) oder (c).

2. DNA gemäß Anspruch 1, dessen mRNA und dessen Genprodukt unter dem Einfluß von Leflunomid verstärkt exprimiert wird.

3. Vektor enthaltend eine DNA nach mindestens einem der Ansprüche 1 und 2.

4. Expressionsvektor nach Anspruch 3 zur Expression des Nukleinsäure-bindenden Proteins mit Helikase- und ATPase-Eigenschaften in einer passenden Wirtszelle.

5. Antisense-Expressionsvektor nach Anspruch 3 zur Expression einer Antisense-RNA, die mit der mRNA hybridisiert, die von der DNA gemäß einem der Ansprüche 1 und 2 kodiert wird.

6. Wirtszelle enthaltend eine DNA nach einem der Ansprüche 1 oder 2 oder einen Vektor nach einem der Ansprüche 3 bis 5.

7. Nukleinsäure-bindendes Protein mit Helikase- und ATPase-Eigenschaften, dessen mRNA und deren Translationsprodukt unter dem Einfluß von Leflunomid verstärkt exprimiert werden, enthaltend die Aminosäuresequenz gemäß Tabelle 2 oder Teile davon mit Helikase- und ATPase-Eigenschaften.

8. Nukleinsäure-bindendes Protein gemäß Anspruch 7, welches aus einer Säugerzellinie stammt.

9. Nukleinsäure-bindendes Protein gemäß Anspruch 7, welches aus einem Abkömmling der murinen Zellinie A20.2J stammt.

10. Verfahren zur Herstellung des Nukleinsäure-bindenden Proteins mit Helikase- und ATPase-Eigenschaften nach einem der Ansprüche 7 bis 9, **dadurch gekennzeichnet, daß**
a) eine Wirtszelle nach Anspruch 6 kultiviert und
b) das Nukleinsäure-bindende Protein mit Helikase- und ATPase-Eigenschaften isoliert wird.

11. Verwendung des Nukleinsäure-bindenden Proteins mit Helikase- und ATPase-Eigenschaften nach einem der Ansprüche 7 bis 9 zur Isolierung spezifisch an dieses Protein bindender RNA's oder Ermittlung ihrer Oligoribonukleotidsequenz.

12. Verwendung des Nukleinsäure-bindenden Proteins mit Helikase- und ATPase-Eigenschaften nach Anspruch 11, **dadurch gekennzeichnet, daß** man
a) besagtes Protein oder Teile davon an eine Matrix koppelt,
b) die so hergestellte Affinitätsmatrix zur Anreicherung von RNA's, die spezifisch an das gekoppelte Protein oder Teile davon binden, aus RNA-Gemischen benutzt.

13. Verwendung des Nukleinsäure-bindenden Proteins mit Helikase- und ATPase-Eigenschaften nach Anspruch 12, **dadurch gekennzeichnet, daß** man zusätzlich zu den Schritten (a) und (b) des Anspruchs 12
c) die so angereicherten RNA-Moleküle mit PCR-Linkem versieht,
d) eine PCR-Anreicherung durchführt, und
e) die so erhaltenen PCR-Fragmente analysiert.

14. Verwendung des Gens des Nukleinsäure-bindenden Proteins mit Helikase- und ATPase-Eigenschaften nach einem der Ansprüche 7 bis 11 als Selektionsmarker bei der Selektion von Zellen gegenüber Leflunomid oder Leflunomidanaloga.

15. Verwendung des Gens des Nukleinsäure-bindenden Proteins mit Helikase- und ATPase-Eigenschaften nach einem der Ansprüche 7 bis 11 als Selektionsmarker als Bestandteil eines Vektors bei der Selektion von Zellen gegenüber Leflunomid oder Leflunomidanaloga.

## Claims

1. Isolated DNA, comprising the gene for a protein which binds nucleic acid and possesses helicase and ATPase properties, selected from the group consisting of:
(a) a DNA containing a DNA sequence as in Table 2;
(b) a DNA containing a DNA sequence which is able to hybridize under stringent conditions with the DNA molecule as in (a);
(c) a DNA containing a DNA sequence which on account of the degeneracy of the genetic code is different from the DNA molecules as in (a) or (b), but makes possible the expression of the proteins which can correspondingly be expressed by the DNA molecules as in (a) and (b);
(d) parts of DNA molecules as in (a), (b) or (c).

2. DNA according to Claim 1, whose mRNA and whose gene product is expressed to an increased extent under the influence of leflunomide.

3. Vector comprising a DNA according to at least one of Claims 1 and 2.

4. Expression vector according to Claim 3 for the expression of the protein which binds nucleic acid and possesses helicase and ATPase properties in a suitable host cell.

5. Antisense expression vector according to Claim 3 for the expression of an antisense RNA, which hybridizes with the mRNA which is encoded by the DNA according to one of Claims 1 and 2.

6. Host cell comprising a DNA according to one of Claims 1 and 2 or a vector according to one of Claims 3 to 5.

7. Protein which binds nucleic acid and possesses helicase and ATPase properties, whose mRNA and whose translation product are expressed to an increased extent under the influence of leflunomide, comprising the amino acid sequence as in Table 2 or parts thereof, possessing helicase and ATPase properties.

8. Protein which binds nucleic acid according to Claim 7, which originates from a mammalian cell line.

9. Protein which binds nucleic acid according to Claim 7, which originates from a derivative of the murine cell line A20.2J.

10. Process for the preparation of the protein which binds nucleic acid and possesses helicase and ATPase properties according to one of Claims 7 to 9, **characterized in that**
a) a host cell according to Claim 6 is cultured and
b) the protein which binds nucleic acid and possesses helicase and ATPase properties is isolated.

11. Use of the protein which binds nucleic acid and possesses helicase and ATPase properties according to one of Claims 7 to 9 for the isolation of RNAs binding specifically to this protein or determination of their oligoribonucleotide sequence.

12. Use of the protein which binds nucleic acid and possesses helicase and ATPase properties according to Claim 11, **characterized in that**
(a) said protein or parts thereof are coupled to a matrix,
(b) the affinity matrix prepared in this way is used for the amplification of RNAs, which bind specifically to the coupled protein or parts thereof, from RNA mixtures.

13. Use of the protein which binds nucleic acid and possesses helicase and ATPase properties according to Claim 12, **characterized in that** additionally to the steps (a) and (b) of claim 12
(c) the RNA molecules amplified in this way are provided with PCR linkers,
(d) a PCR amplification is carried out, and
(e) the PCR fragments thus obtained are analysed.

14. Use of the gene of the protein which binds nucleic acid and possesses helicase and ATPase properties according to one of Claims 7 to 11 as a selection marker in the selection of cells against lefluonomide or leflunomide analogues.

15. Use of the gene of the protein which binds nucleic acid and possesses helicase and ATPase properties according to one of Claims 7 to 11 as a selection marker as a constituent of a vector in the selection of cells against leflunomide or leflunomide analogues.

## Revendications

1. ADN isolé comprenant le gène codant pour une protéine de liaison aux acides nucléiques présentant des propriétés hélicase et ATPase, choisi dans le groupe constitué de :
a) un ADN comprenant une séquence d'ADN selon le Tableau 2 ;
b) un ADN comprenant une séquence d'ADN qui est capable de s'hybrider à la molécule d'ADN selon (a) dans des conditions stringentes ;
c) un ADN comprenant une séquence d'ADN, qui est différente des molécules d'ADN selon (a) et (b) en raison de la dégénérescence du code génétique, mais permet cependant l'expression des protéines exprimables correspondant aux molécules d'ADN selon (a) et (b) ;
d) des fragments de molécules d'ADN selon (a), (b) ou (c).

2. ADN selon la revendication 1, dont l'ARNm et le produit du gène est davantage exprimé sous l'influence du Leflunomid.

3. Vecteur comprenant un ADN selon au moins l'une des revendications 1 et 2.

4. Vecteur d'expression selon la revendication 3, pour l'expression de la protéine de liaison aux acides nucléiques présentant des propriétés hélicase et ATPase dans une cellule hôte appropriée.

5. Vecteur d'expression antisens selon la revendication 3, pour l'expression d'un ARN antisens qui s'hybride avec l'ARNm codé à partir de l'ADN selon l'une des revendications 1 et 2.

6. Cellule hôte comprenant un ADN selon l'une des revendications 1 ou 2 ou un vecteur selon l'une des revendications 3 à 5.

7. Protéine de liaison aux acides nucléiques présentant des propriétés hélicase et ATPase, dont l'ARNm et le produit de la traduction sont davantage exprimés sous l'influence du Leflunomid, comprenant la séquence en acides aminés selon le Tableau 2 ou des fragments de celle-ci présentant des propriétés hélicase et ATPase.

8. Protéine de liaison aux acides nucléiques selon la revendication 7, qui provient d'une lignée cellulaire de mammifère.

9. Protéine de liaison aux acides nucléiques selon la revendication 7, qui provient d'un descendant de la lignée cellulaire murine A20.2J.

10. Procédé de préparation d'une protéine de liaison aux acides nucléiques présentant des propriétés hélicase et ATPase selon l'une des revendications 7 à 9, **caractérisé en ce que** l'on
a) cultive une cellule hôte selon la revendication 6 et
b) isole la protéine de liaison aux acides nucléiques présentant des propriétés hélicase et ATPase.

11. Utilisation de la protéine de liaison aux acides nucléiques présentant des propriétés hélicase et ATPase selon l'une des revendications 7 à 9 pour isoler spécifiquement les ARN liés à cette protéine ou déterminer leur séquence oligoribonucléotidique.

12. Utilisation de la protéine de liaison aux acides nucléiques présentant des propriétés hélicase et ATPase selon la revendication 11, **caractérisée en ce que** l'on
a) couple ladite protéine ou des fragments de celle-ci à une matrice,
b) utilise la matrice d'affinité ainsi préparée pour enrichir, à partir de mélanges d'ARN, en ARN qui se lient spécifiquement à la protéine couplée ou des fragments de celle-ci.

13. Utilisation de la protéine de liaison aux acides nucléiques présentant des propriétés hélicase et ATPase selon la revendication 12, **caractérisée en ce que**, en plus des étapes (a) et (b) de la revendication 12, on
c) apporte les molécules d'ARN ainsi enrichies à des amorces de PCR
d) met en oeuvre un enrichissement par PCR, et
e) analyse les fragments de PCR ainsi obtenus.

14. Utilisation du gène de la protéine de liaison aux acides nucléiques présentant des propriétés hélicase et ATPase selon l'une des revendications 7 à 11, comme marqueur de sélection pour la sélection de cellules contre le Leflunomid ou des analogues du Leflunomid.

15. Utilisation du gène de la protéine de liaison aux acides nucléiques présentant des propriétés hélicase et ATPase selon l'une des revendications 7 à 11, comme marqueur de sélection comme composant d'un vecteur pour la sélection de cellules contre le Leflunomid ou des analogues du Leflunomid.


### SEQUENZPROTOKOLL

(1) ALLGEMEINE INFORMATION:
   (i) ANMELDER:
      (A) NAME: Hoechst Aktiengesellschaft
      (B) STRASSE: -
      (C) ORT: Frankfurt
      (D) BUNDESLAND: -
      (E) LAND: Deutschland
      (F) POSTLEITZAHL: 65926
      (G) TELEPHON: 069-305-3005
      (H) TELEFAX: 069-35-7175
      (I) TELEX: 4 1 234 700 ho d
   (ii) ANMELDETITEL: ATP- und Nukleinsaeure-bindendes Protein mit putativen Helikase- und ATPase-Eigenschaften
   (iii) ANZAHL DER SEQUENZEN: 18
   (iv) COMPUTER-LESBARE FORM:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.25 (EPA)
(2) INFORMATION ZU SEQ ID NO: 1:
   (i) SEQUENZ CHARAKTERISTIKA:
      (A) LÄNGE: 11 Aminosäuren
      (B) ART: Aminosäure
      (C) STRANGFORM: Einzel
      (D) TOPOLOGIE: linear
   (ii) ART DES MOLEKÜLS: Peptid
   (ix) MERKMALE:
      (A) NAME/SCHLÜSSEL: Peptide
      (B) LAGE: 1..11
   (xi) SEQUENZBESCHREIBUNG: SEQ ID NO: 1:
   Lys Leu Gly Asp Ile Met Gly Val Lys Lys Glu
   1               5                   10
(2) INFORMATION ZU SEQ ID NO: 2:
   (i) SEQUENZ CHARAKTERISTIKA:
      (A) LÄNGE: 16 Aminosäuren
      (B) ART: Aminosäure
      (C) STRANGFORM: Einzel
      (D) TOPOLOGIE: linear
   (ii) ART DES MOLEKÜLS: Peptid
   (ix) MERKMALE:
      (A) NAME SCHLÜSSEL: Peptide
      (B) LAGE: 1..16
   (xi) SEQUENZBESCHREIBUNG: SEQ ID NO: 2:
   Lys Leu Gly Asp Ile Met Gly Val Lys Lys Glu Thr Glu Pro Asp Lys
   1               5                   10                  15
(2) INFORMATION ZU SEQ ID NO: 3:
   (i) SEQUENZ CHARAKTERISTIKA:
      (A) LÄNGE: 13 Aminosäuren
      (B) ART: Aminosäure
      (C) STRANGFORM: Einzel
      (D) TOPOLOGIE: linear
   (ii) ART DES MOLEKÜLS: Peptid
   (ix) MERKMALE:
      (A) NAME/SCHLÜSSEL: Peptide
      (B) LAGE: 1..13
   (xi) SEQUENZBESCHREIBUNG: SEQ ID NO: 3:
   Lys Leu Ile Val Thr Ser Ala Thr Met Asp Ala Glu Lys
   1               5                   10
(2) INFORMATION ZU SEQ ID NO: 4:
   (i) SEQUENZ CHARAKTERISTIKA:
      (A) LÄNGE: 12 Aminosäuren
      (B) ART: Aminosäure
      (C) STRANGFORM: Einzel
      (D) TOPOLOGIE: linear
   (ii) ART DES MOLEKÜLS: Peptid
   (ix) MERKMALE:
      (A) NAME/SCHLÜSSEL: Peptide
      (B) LAGE: 1..12
   (xi) SEQUENZBESCHREIBUNG: SEQ ID NO: 4:
   Asp Ala Thr Ser Asp Leu Ala Ile Ile Ala Arg Lys
   1               5                   10
(2) INFORMATION ZU SEQ ID NO: 5:
   (i) SEQUENZ CHARAKTERISTIKA:
      (A) LÄNGE: 5 Aminosäuren
      (B) ART: Aminosäure
      (C) STRANGFORM: Einzel
      (D) TOPCLOGIE: linear
   (ii) ART DES MOLEKÜLS: Peptid
   (ix) MERKMALE:
      (A) NAME SCHLÜSSEL: Peptide
      (B) LAGE: 1..5
   (xi) SEQL1ENZBESCHREIBUNG: SEQ ID NO: 5:
(2) INFORMATION ZU SEQ ID NO: 6:
   (i) SEQUENZ CHARAKTERISTIKA:
      (A) LÄNGE: 11 Aminosäuren
      (B) ART: Aminosäure
      (C) STRANGFORM: Einzel
      (D) TOPOLOGIE: linear
   (ii) ART DES MOLEKÜLS: Peptid
   (ix) MERKMALE:
      (A) NAME/SCHLÜSSEL: Peptide
      (B) LAGE: 1..11
   (xi) SEQUENZBESCHREIBUNG: SEQ ID NO: 6:
   Thr Pro Gln Glu Asp Tyr Val Glu Ala Ala Val
   1               5                   10
(2) INFORMATION ZU SEQ ID NO: 7:
   (i) SEQUENZ CHARAKTERISTIKA:
      (A) LÄNGE: 17 Basenpaare
      (B) ART: Nukleinsäure
      (C) STRANGFORM: Einzel
      (D) TOPOLOGIE: linear
   (ii) ART DES MOLEKÜLS: DNS (genomisch)
   (ix) MERKMALE:
      (A) NAME/SCHLÜSSEL: exon
      (B) LAGE: 1..17
   (xi) SEQUENZBESCHREIBUNG: SEQ ID NO: 7:
      ATGGGNGTNA ARAARGG 17
(2) INFORMATION ZU SEQ ID NO: 8:
   (i) SEQUENZ CHARAKTERISTIKA:
      (A) LÄNGE: 17 Basenpaare
      (B) ART: Nukleinsäure
      (C) STRANGFORM: Einzel
      (D) TOPOLOGIE: linear
   (ii) ART DES MOLEKÜLS: ONS (genomisch)
   (ix) MERKMALE:
      (A) NAME SCHLÜSSEL: exon
      (B) LAGE: 1..17
   (xi) SEQUENZBESCHREIBUNG: SEQ ID NO: 8:
      GATATYATSC GNGTNAA 17
(2) INFORMATION ZU SEQ ID NO: 9:
   (i) SEQUENZ CHARAKTERISTIKA:
      (A) LÄNGE: 20 Basenpaare
      (B) ART: Nukleinsäure
      (C) STRANGFORM: Einzel
      (D) TOPOLOGIE: linear
   (ii) ART DES MOLEKÜLS: DNS (genomisch)
   (ix) MERKMALE:
      (A) NAME/SCHLÜSSEL: exon
      (B) LAGE: 1..20
   (xi) SEQUENZBESCHREIBUNG: SEQ ID NO: 9:
      ATGGTNGTNA ARAARGARAC 20
(2) INFORMATION ZU SEQ ID NO: 10:
   (i) SEQUENZ CHARAKTERISTIKA:
      (A) LÄNGE: 20 Basenpaare
      (B) ART: Nukleinsäure
      (C) STRANGFORM: Einzel
      (D) TOPOLOGIE: linear
   (ii) ART DES MOLEKÜLS: DNS (genomisch)
   (ix) MERKMALE:
      (A) NAME/SCHLÜSSEL: exon
      (B) LAGE: 1..20
   (xi) SEQUENZBESCHREIBUNG: SEQ ID NO: 10:
      AARGARACNG ARCCNGAYAA 20
(2) INFORMATION ZU SEQ ID NO: 11:
   (i) SEQUENZ CHARAKTERISTIKA:
      (A) LÄNGE: 18 Basenpaare
      (B) ART: Nukleinsäure
      (C) STRANGFORM: Einzel
      (D) TOPOLOGIE: linear
   (ii) ART DES MOLEKÜLS: DNS (genomisch)
   (ix) MERKMALE:
      (A) NAME SCHLÜSSEL: exon
      (B) LAGE: 1..18
   (xi) SEQUENZBESCHREIBUNG: SEQ ID NO: 11:
      RTCCATNGTN GCNGANGT 18
(2) INFORMATION ZU SEQ ID NO: 12:
   (i) SEQUENZ CHARAKTERISTIKA:
      (A) LÄNGE: 18 Basenpaare
      (B) ART: Nukleinsäure
      (C) STRANGFORM: Einzel
      (D) TOPOLOGIE: linear
   (ii) ART DES MOLEKÜLS: DNS (genomisch)
   (ix) MERKMALE:
      (A) NAME/SCHLÜSSEL: exon
      (B) LAGE: 1..18
   (xi) SEQUENZBESCHREIBUNG: SEQ ID NO: 12:
      NGTAGCNGAN GTNACNAT 18
(2) INFORMATION ZU SEQ ID NO: 13:
   (i) SEQUENZ CHARAKTERISTIKA:
      (A) LÄNGE: 27 Basenpaare
      (B) ART: Nukleinsäure
      (C) STRANGFORM: Einzel
      (D) TOPOLOGIE: linear
   (ii) ART DES MOLEKÜLS: DNS (genomisch)
   (ix) MERKMALE:
      (A) NAME/SCHLÜSSEL: exon
      (B) LAGE: 1..27
   (xi) SEQUENZBESCHREIBUNG: SEQ ID NO: 13:
      TGTGATCTGC AAACATCTGC ACTGTCC 27
(2) INFORMATION ZU SEQ ID NO: 14:
   (i) SEQUENZ CHARAKTERISTIKA:
      (A) LÄNGE: 27 Basenpaare
      (B) ART: Nukleinsäure
      (C) STRANGFORM: Einzel
      (D) TOPOLOGIE: linear
   (ii) ART DES MOLEKÜLS: DNS (genomisch)
   (ix) MERKMALE:
      (A) NAME/SCHLÜSSEL: exon
      (B) LAGE: 1..27
   (xi) SEQUENZBESCHREIBUNG: SEQ ID NO: 14:
      GCCGGTGATT GCCAGTGAAG GATGCCA 27
(2) INFORMATION ZU SEQ ID NO: 15:
   (i) SEQUENZ CHARAKTERISTIKA:
      (A) LÄNGE: 612 Basenpaare
      (B) ART: Nukleinsäure
      (C) STRANGFORM: Doppel
      (D) TOPOLOGIE: linear
   (ii) ART DES MOLEKÜLS: DNS (genomisch)
   (ix) MERKMALE:
      (A) NAME/SCHLÜSSEL: exon
      (B) LAGE: 1..612
   (xi) SEQUENZBESCHREIBUNG: SEQ ID NO: 15:
   AAGGAGACGG AGCCGGACAA AGCTATGACA GAAGACGGGA AAGTGGACTA CAGGACGGAG     60
   CAGAAGTTTG CAGATCACAT GAAGGAGAAA AGCGAGGCCA GCAGTGAGTT TGCCAAGAAG    120
   AAGTCGATCC TGGAGCAGAG GCAGTACCTG CCCATCTTTG CCGTGCAGCA GGAGCTCGTC    180
   ACCATCATCA GAGACAACAG CATTGTGGTC GTGGTCGGGG AGACAGGGAG TGGCAAGACC    240
   ACTCAGCTGA CCCAGTACTT GCATGAAGAT GGTTACACGG ACTATGGGAT GATCGGGTGT    300
   ACCCAGCCCC GGCGTGTGGC TGCCATGTCA GCGGCCAAGA GAGTCAGTGA AGAGATGGGG    360
   GGCAACCTTG GAGAAGAGGT GGGCTATGCC ATCCGCTTTG AGGACTGCAC TTCGGAAAAC    420
   AACTTGATCA AGTACATGAC GGATGGGATC CTGCTGCGCG AGTCCCTCCG GCAGGCTGAC    480
   CTGGACCACT ACAGCGCCGT CATCATGGAT GAGGCCCACG AGCGCTCCCT CAACACCGAC    540
   GTGCTTTTTG GGCTGCTCCG GGAGGTTGTG GCTCGAGGCT CAGACCTGAA GCTCATGGTT    600
   ACATCGGCTA CT                                                        612
(2) INFORMATION ZU SEQ ID NO: 16:
   (i) SEQUENZ CHARAKTERISTIKA:
      (A) LÄNGE: 204 Aminosäuren
      (B) ART: Aminosäure
      (C) STRANGFORM: Einzel
      (D) TOPOLOGIE: linear
   (ii) ART DES MOLEKÜLS: Protein
   (ix) MERKMALE:
      (A) NAME/SCHLÜSSEL: Protein
      (B) LAGE: 1..204
   (xi) SEQUENZBESCHREIBUNG: SEQ ID NO: 16:
   Lys Glu Thr Glu Pro Asp Lys Ala Met Thr Glu Asp Gly Lys Val Asp
   1               5                   10                  15
   Tyr Arg Thr Glu Gln Lys Phe Ala Asp His Met Lys Glu Lys Ser Glu
               20                  25                  30
   Ala Ser Ser Glu Phe Ala Lys Lys Lys Ser Ile Leu Glu Gln Arg Gln
           35                  40                  45
   Tyr Leu Pro Ile Phe Ala Val Gln Gln Glu Leu Val Thr Ile Ile Arg
       50                  55                  60
   Asp Asn Ser Ile Val Val Val Val Gly Glu Thr Gly Ser Gly Lys Thr
   65                  70                  75                  80
   Thr Gln Leu Thr Gln Tyr Leu His Glu Asp Gly Tyr Thr Asp Tyr Gly
                   85                  90                  95
   Met Ile Gly Cys Thr Gln Pro Arg Arg Val Ala Ala Met Ser Ala Ala
               100                 105                 110
   Lys Arg Val Ser Glu Glu Met Gly Gly Asn Leu Gly Glu Glu Val Gly
           115                 120                 125
   Tyr Ala Ile Arg Phe Glu Asp Cys Thr Ser Glu Asn Asn Leu Ile Lys
       130                 135                 140
   Tyr Met Thr Asp Gly Ile Leu Leu Arg Glu Ser Leu Arg Gln Ala Asp
   145                 150                 155                 160
   Leu Asp His Tyr Ser Ala Val Ile Met Asp Glu Ala His Glu Arg Ser
                   165                 170                 175
   Leu Asn Thr Asp Val Leu Phe Gly Leu Leu Arg Glu Val Val Ala Arg
               180                 185                 190
   Gly Ser Asp Leu Lys Leu Met Val Thr Ser Ala Thr
           195                 200
(2) INFORMATION ZU SEQ ID NO: 17:
   (i) SEQUENZ CHARAKTERISTIKA:
      (A) LÄNGE: 3684 Basenpaare
      (B) ART: Nukleinsäure
      (C) STRANGFORM: Doppel
      (D) TOPOLOGIE: linear
   (ii) ART DES MOLEKÜLS: DNS (genomisch)
   (ix) MERKMALE:
      (A) NAME/SCHLÜSSEL: exon
      (B) LAGE: 1..3684
   (xi) SEQUENZBESCHREIBUNG: SEQ ID NO: 17:
   ATGGGGGACA CCAGTGAGGA TGCCTCGATC CATCGATTGG AAGGCACTGA TCTGGACTGT    60
   CAGGTTGGTG GTCTTATTTG CAAGTCCAAA AGTGCGGCCA GCGAGCAGCA TGTCTTCAAG   120
   GCTCCTGCTC CCCGCCCTTC ATTACTCGGA CTGGACTTGC TGGCTTCCCT GAAACGGAGA   180
   GAGCGAGAGG AGAAGGACGA TGGGGAGGAC AAGAAGAAGT CCAAAGTCTC CTCCTACAAG   240
   GACTGGGAAG AGAGCAAGGA TGACCAGAAG GATGCTGAGG AAGAGGGCGG TGACCAGGCT   300
   GGCCAAAATA TCCGGAAAGA CAGACATTAT CGGTCTGCTC GGGTAGAGAC TCCATCCCAT   360
   CCGGGTGGTG TGAGCGAAGA GTTTTGGGAA CGCAGTCGGC AGAGAGAGCG GGAGCGGCGG   420
   GAACATGGTG TCTATGCCTC GTCCAAAGAA GAAAAGGATT GGAAGAAGGA GAAATCGCGG   480
   GATCGAGACT ATGACCGCAA GAGGGACAGA GATGAGCGGG ATAGAAGTAG GCACAGCAGC   540
   AGATCAGAGC GAGATGGAGG GTCAGAGCGT AGCAGCAGAA GAAATGAACC CGAGAGCCCA     600
   CGACATCGAC CTAAAGATGC AGCCACCCCT TCAAGGTCTA CCTGGGAGGA AGAGGACAGT     660
   GGCTATGGCT CCTCAAGGCG CTCACAGTGG GAATCGCCCT CCCCGACGCC TTCCTATCGG     720
   GATTCTGAGC GGAGCCATCG GCTGTCCACT CGAGATCGAG ACAGGTCTGT GAGGGGCAAG     780
   TACTCGGATG ACACGCCTCT GCCAACTCCC TCCTACAAAT ATAACGAGTG GGCCGATGAC     840
   AGAAGACACT TGGGGTCCAC CCCGCGTCTG TCCAGGGGCC GAGGAAGACG TGAGGAGGGC     900
   GAAGAAGGAA TTTCATTTGA CACGGAGGAG GAGCGGCAGC AGTGGGAAGA TGACCAGAGG     960
   CAAGCCGATC GGGATTGGTA CATGATGGAC GAGGGCTATG ACGAGTTCCA CAACCCGCTG    1020
   GCCTACTCCT CCGAGGACTA CGTGAGGAGG CGGGAGCAGC ACCTGCATAA ACAGAAGCAG    1080
   AAGCGCATTT CAGCTCAGCG GAGACAGATC AATGAGGATA ACGAGCGCTG GGAGACAAAC    1140
   CGCATGCTCA CCAGTGGGGT GGTCCATCGG CTGGAGGTGG ATGAGGACTT TGAAGAGGAC    1200
   AACGCGGCCA AGGTGCATCT GATGGTGCAC AATCTGGTGC CTCCCTTTCT GGATGGGCGC    1260
   ATTGTCTTCA CCAAGCAGCC GGAGCCGGTG ATTCCAGTGA AGGATGCTAC TTCTGACCTG    1320
   GCCATCATTG CTCGGAAAGG CAGCCAGACA GTGCGGAAGC ACAGGGAGCA GAAGGAGCGC    1380
   AAGAAGGCTC AGCACAAACA CTGGGAACTG GCGGGGACCA AACTGGGAGA TATAATGGGC    1440
   GTCAAGAAGG AGGAAGAGCC AGATAAAGCT GTGACGGAGG ATGGGAAGGT GGACTACAGG    1500
   ACAGAGCAGA AGTTTGCAGA TCACATGAAG AGAAAGAGCG AAGCCAGCAG TGAATTTGCA    1560
   AAGAAGAAGT CCATCCTGGA GCAGAGGCAG TACCTGCCCA TCTTTGCAGT GCAGCAGGAG    1620
   CTGCTCACTA TTATCAGAGA CAACAGCATC GTGATCGTGG TTGGGGAGAC GGGGAGTGGT    1680
   AAGACCACTC AGCTGACGCA GTACCTGCAT GAAGATGGTT ACACGGACTA TGGGATGATT    1740
   GGGTGTACCC AGCCCCGGCG TGTAGCTGCC ATGTCAGTGG CCAAGAGAGT CAGTGAAGAG    1800
   ATGGGGGGAA ACCTTGGCGA GGAGGTGGGC TATGCCATCC GCTTTGAAGA CTGCACTTCA    1860
   GAGAACACCT TGATCAAATA CATGACTGAC GGGATCCTGC TCCGAGAGTC CCTCCGGGAA    1920
   GCCGACCTGG ATCACTACAG TGCCATCATC ATGGACGAGG CCCACGAGCG CTCCCTCAAC    1980
   ACTGACGTGC TCTTTGGGCT GCTCCGGGAG GTAGTGGCTC GGCGCTCAGA CCTGAAGCTC    2040
   ATCGTCACAT CAGCCACGAT GGATGCGGAG AAGTTTGCTG CCTTTTTTGG GAATGTCCCC    2100
   ATCTTCCACA TCCCTGGCCG TACCTTCCCT GTTGACATCC TCTTCAGCAA GACCCCACAG    2160
   GAGGATTACG TGGAGGCTGC AGTGAAGCAG TCCTTGCAGG TGCACCTGTC GGGGGCCCCT    2220
   GGAGACATCC TTATCTTCAT GCCTGGCCAA GAGGACATTG AGGTGACCTC AGACCAGATT    2280
   GTGGAGCATC TGGAGGAACT GGAGAACGCG CCTGCCCTGG CTGTGCTGCC CATCTACTCT    2340
   CAGCTGCCTT CTGACCTCCA GGCCAAAATC TTCCAGAAGG CTCCAGATGG CGTTCGGAAG    2400
   TGCATCGTTG CCACCAATAT TGCCGAGACG TCTCTCACTG TTGACGGCAT CATGTTTGTT    2460
   ATCGATTCTG GTTATTGCAA ATTAAAGGTC TTCAACCCCA GGATTGGCAT GGATGCTCTG    2520
   CAGATCTATC CCATTAGCCA GGCCAATGCC AACCAGCGGT CAGGGCGAGC CGGCAGGACG    2580
   GGCCCAGGTC AGTGTTTCAG GCTCTACACC CAGAGCGCCT ACAAGAATGA GCTCCTGACC     2640
   ACCACAGTGC CCGAGATCCA GAGGACTAAC CTGGCCAACG TGGTGCTGCT GCTCAAGTCC     2700
   CTCGGGGTGC AGGACCTGCT GCAGTTCCAC TTCATGGACC CGCCCCCGGA GGACAACATG     2760
   CTCAACTCTA TGTATCAGCT CTGGATCCTC GGGGCCCTGG ACAACACAGG TGGTCTGACC     2820
   TCTACCGGGC GGCTGATGGT GGAGTTCCCG CTGGACCCTG CCCTGTCCAA GATGCTCATC     2880
   GTGTCCTGTG ACATGGGCTG CAGCTCCGAG ATCCTGCTCA TCGTTTCCAT GCTCTCGGTC     2940
   CCAGCCATCT TCTACAGGCC CAAGGGTCGA GAGGAGGAGA GTGATCAAAT CCGGGAGAAG     3000
   TTCGCTGTTC CTGAGAGCGA TCATTTGACC TACCTGAATG TTTACCTGCA GTGGAAGAAC     3060
   AATAATTACT CCACCATCTG GTGTAACGAT CATTTCATCC ATGCTAAGGC CATGCGGAAG     3120
   GTCCGGGAGG TGCGAGCTCA ACTCAAGGAC ATCATGGTGC AGCAGCGGAT GAGCCTGGCC     3180
   TCGTGTGGCA CTGACTGGGA CATCGTCAGG AAGTGCATCT GTGCTGCCTA TTTCCACCAA     3240
   GCAGCCAAGC TCAAGGGAAT CGGGGAGTAC GTGAACATCC GCACAGGGAT GCCCTGCCAC     3300
   TTGCACCCCA CCAGCTCCCT TTTTGGAATG GGCTACACCC CAGATTACAT AGTGTATCAC     3360
   GAGTTGGTCA TGACCACCAA GGAGTATATG CAGTGTGTGA CCGCTGTGGA CGGGGAGTGG     3420
   CTGGCGGAGC TGGGCCCCAT GTTCTATAGC GTGAAACAGG CGGGCAAGTC ACGGCAGGAG     3480
   AACCGTCGTC GGGCCAAAGA GGAAGCCTCT GCCATGGAGG AGGAGATGGC GCTGGCCGAG     3540
   GAGCAGCTGC GAGCCCGGCG GCAGGAGCAG GAGAAGCGCA GCCCCCTGGG CAGTGTCAGG     3600
   TCTACGAAGA TCTACACTCC AGGCCGGAAA GAGCAAGGGG AGCCCATGAC CCCTCGCCGC     3660
   ACGGCAGCCC GCTTTGGTCT GTGA                                            3684
(2) INFORMATION ZU SEQ ID NO: 18:
   (i) SEQUENZ CHARAKTERISTIKA:
      (A) LÄNGE: 1227 Aminosäuren
      (B) ART: Aminosäure
      (C) STRANGFORM: Einzel
      (D) TOPOLOGIE: linear
   (ii) ART DES MOLEKÜLS: Protein
   (ix) MERKMALE:
      (A) NAME/SCHLÜSSEL: Protein
      (B) LAGE: 1..1227
   (xi) SEQUENZBESCHREIBUNG: SEQ ID NO: 18: